# EUROPEAN PATENT SPECIFICATION

(11) **EP 0 797 436 B1**
(45) Date of publication and mention of the grant of the patent: **15.03.2006**
(21) Application number: 95939149.1
(22) Date of filing: 03.11.1995
(51) Int. Cl.: A61K 48/00

(54) **NOVEL ADENOVIRAL VECTORS, PACKAGING CELL LINES, RECOMBINANT ADENOVIRUSES AND METHODS**
NEUARTIGE ADENOVIRALE VEKTOREN, VERPACKUNGSSZELLINIEN, REKOMBINANTE ADENOVIREN UND VERFAHREN
NOUVEAUX VECTEURS ADENOVIRAUX, LIGNEES CELLULAIRES D'ENCAPSIDATION, ADENOVIRUS RECOMBINES ET PROCEDES

(30) Priority: 03.11.1994 US 333680
(43) Date of publication of application: 01.10.1997
(73) Proprietor: CELL GENESYS, INC., Foster City, CA 94404 (US)
(72) Inventor: WANG, Qing, Palo Alto, CA 94303 (US); FINER, Mitchell, H., San Carlos, CA 94070 (US); JIA, Xiao-Chi, San Mateo, CA 94403 (US)
(74) Representative: Horner, Martin Grenville
(86) International application number: PCT/US1995/014793
(87) International publication number: WO 1996/014061

(56) References cited:
- WO-A-94/12649
- WO-A-94/28152
- WO-A-95/27071
- US-A- 5 173 414
- US-A- 5 252 479
- US-A- 5 354 678
- CURRENT OPINION IN GENETICS AND DEVELOPMENT, Volume 3, issued 1993, KOZARSKY et al., "Gene Therapy: Adenovirus Vectors", pages 499-503.
- ADVANCED DRUG DELIVERY REVIEWS, Volume 12, issued 1993, XIAO et al., "Adeno-Associated Virus (AAV) Vectors for Gene Transfer", pages 201-215.
- CURRENT OPINION IN BIOTECHNOLOGY, Volume 3, issued 1992, CARTER, "Adeno-Associated Virus Vectors", pages 533-539.
- HUMAN GENE THERAPY, Volume 5, issued 1994, KOTIN R.M., "Prospects for the Use of Adeno-Associated Virus as a Vector for Human Gene Therapy", pages 793-801.
- BIOTECHNOLOGY, Volume 20, issued 1992, GRAHAM et al., "Adenovirus-Based Expression Vectors and Recombinant Vaccines", pages 363-390.
- BIOTECHNIQUES, Volume 6, issued 1989, BERKNER, "Development of Adenovirus Vectors for the Expression of Heterologous Genes", pages 616-629.
- TRENDS IN CARDIOVASCULAR MEDICINE, Volume 3, No. 5, issued 1993, GERARD et al., "Adenovirus-Mediated Gene Transfer", pages 171-177.
- ADVANCED DRUG DELIVERY REVIEWS, Volume 12, issued 1993, TRAPNELL, "Adenoviral Vectors for Gene Transfer", pages 185-199.

## Description

### FIELD OF THE INVENTION

The present invention relates to novel replication-deficient adenoviral vectors and novel packaging cell lines. In particular, the novel packaging cell lines have the complementary function for the early gene region E1, E4 and optionally the E3 deletions of human adenovirus.

### BACKGROUND OF THE INVENTION

Replication-defective retroviral vectors as gene transfer vehicles provide the foundation for human gene therapy. Retroviral vectors are engineered by removing or altering all viral genes so that no viral proteins are made in cells infected with the vector and further virus spread occurs. The development of packaging cell lines which are required for the propagation of retroviral vectors were the most important step toward the reality of human gene therapy. The foremost advantages of retroviral vectors for gene therapy are the high efficiency of gene transfer and the precise integration of the transferred genes into cellular genomic DNA. However, major disadvantages are also associated with retroviral vectors, namely, the inability of retroviral vectors to transduce non-dividing cells and the potential insertional mutagenesis.

Human adenoviruses have been developed as live viral vaccines and provide another alternative for *in vivo* gene delivery vehicles for human gene therapy [Graham & Prevec in New Approaches to Immunological Problems, Ellis (ed), Butterworth-Heinemann, Boston, MA, pp. 363-390 (1992) Rosenfeld, et al, *Science* **252**: 431-434 (1991), Rosenfeld, et al, *Cell* **68:** 143-155 (1992), and Ragot, et al, Nature **361**: 647-650 (1993)]. The features which make recombinant adenoviruses potentially powerful gene delivery vectors have been extensively reviewed [Berkner, *Biotechniques 6:* 616-629, (1988) and Kozarsky & Wilson, *Curr. Opin. Genet. Dev.* **3**: 499-503, (1993)]. Briefly, recombinant adenoviruses can be grown and purified in large quantities and efficiently infect a wide spectrum of dividing and non-dividing mammalian cells *in vivo.* Moreover, the adenoviral genome may be manipulated with relative ease and accommodate very large insertions of DNA.

The first generation of recombinant adenoviral vectors currently available have a deletion in the viral early gene region 1 (herein called E1 which comprises the E1a and E1b regions from genetic map units 1.30 to 9.24) which for most uses is replaced by a transgene. A transgene is a heterologous or foreign (exogenous) gene that is carried by a viral vector and transduced into a host cell. Deletion of the viral E1 region renders the recombinant adenovirus defective for replication and incapable of producing infectious viral particles in the subsequently infected target cells [Berkner, *Biotechniques* **6**: 616-629 (1988)]. The ability to generate E1-deleted adenoviruses is based on the availability of the human embryonic kidney packaging cell line called 293. This cell line contains the E1 region of the adenovirus which provides the E1 region gene products lacking in the El-deleted virus [Graham, et al, *J. Gen Virol.* **36:** 59-72, (1977)]. However, the inherent flaws of current first generation recombinant adenoviruses have drawn increasing concerns about its eventual usage in patients. Several recent studies have shown that E1 deleted adenoviruses are not completely replication incompetent [Rich, *Hum*. *Gene. Ther.* **4**: 461-476 (1993) and Engelhardt, et al, *Nature Genet*. **4**: 27-34 (1993)]. Three general limitations are associated with the adenoviral vector technology. First, infection both *in vivo* and *in vitro* with the adenoviral vector at high multiplicity of infection (abbreviated m.o.i.) has resulted in cytotoxicity to the target cells, due to the accumulation of penton protein, which is itself toxic to mammalian cells [(Kay, *Cell Biochem*. **17E:** 207 (1993)]. Second, host immune responses against adenoviral late gene products, including penton protein, cause the inflammatory response and destruction of the infected tissue which received the vectors [Yang, et al, *Proc. Natl, Acad. Sci. USA* **91**: 4407-4411 (1994)]. Lastly, host immune responses and cytotoxic effects together prevent the long term expression of transgenes and cause decreased levels of gene expression following subsequent administration of adenoviral vectors [Mittal, et al, *Virus Res*.**28**: 67-90 (1993)].

In view of these obstacles, further alterations in the adenoviral vector design are required to cripple the ability of the virus to express late viral gene proteins, decreasing host cytotoxic responses and the expectation of decreasing host immune response. Engelhardt et al recently constructed a temperature sensitive (ts) mutation within the E2A-encoded DNA-binding protein (DBP) region of the El-deleted recombinant adenoviral vector [Engelhardt, et al, *Proc. Natl. Acad. Sci. USA* **91:** 6196-6200 (1994)] which fails to express late gene products at non-permissive temperatures *in vitro*. Diminished inflammatory responses and prolonged transgene expression were reported in animal livers infected by this vector (Engelhart, et al 1994). However, the ts DBP mutation may not give rise to a full inactive gene product in vivo, and therefore be incapable of completely blocking late gene expression. Further technical advances are needed that would introduce a second lethal deletion into the adenoviral El-deleted vectors to completely block late gene expression *in vivo*. Novel packaging cell lines that can accommodate the production of second (and third) generation recombinant adenoviruses rendered replication-defective by the deletion of the E1 and E4 gene regions hold the greatest promise towards the development of safe and efficient vectors for human gene therapy. The present invention provides for such packaging cell lines and resultant mutant viruses and recombinant viral vectors (for example, adenoviral or AAV-derived) carrying the transgene of interest.

### SUMMARY OF THE INVENTION

Accordingly, the present invention generally aims to provide an improved adenoviral vector system to obviate the difficulties found in using the first generation adenoviral vectors currently available by providing second and third generation viral vectors deleted of at least two early region DNA sequences, and that are capable of delivering foreign, therapeutic or transgenes to somatic cells.

In particular, the present invention provides for second and third generation recombinant adenoviral vectors (adenoviruses) harboring at least two lethal deletions, namely, the E1 and E4 early region genes. Optionally, this vector may also be deleted of the E3 early gene region. More particularly, this recombinant viral vector carries a transgene, for example, the β-galactosidase gene, introduced into either the E1 or E4 regions. In a more particular embodiment, the recombinant adenoviruses may contain a therapeutic gene that replaces the E1 or E4 regions (or optionally the E3 region), and the therapeutic gene is expressed and/or transcribed in a targeted host cell.

Another object of the present invention is to provide a novel packaging cell line which complements functions of the E1, E4 and optionally the E3 gene regions of a defective adenovirus deleted of the E1, E4 and optionally E3 regions, thereby allowing the production of the above described second generation recombinant adenoviral vectors deficient of the E1, E4 and optionally, the E3 DNA regions. The preferred packaging cell line derived from human embryonic kidney cells (293 cell line) contains the adenovirus E1 and E4 gene regions integrated into its genome. In a particular embodiment, the packaging cell line is identified herein as 293-E4 and deposited on August 30, 1994, with the American Type Culture Collection (ATCC), 12301 Parklawn Drive, Rockville, Maryland, under the Budapest Treaty, and has there been designated ATCC # CRL 11711.

Another object of the present invention is to provide a novel packaging cell line which complements functions of the E1, E4 and optionally the E3 gene regions of a defective adenovirus deleted of the E1, E4 and optionally E3 regions, thereby allowing the production of the above described second generation recombinant adenoviral vectors deficient of the E1, E4 and optionally, the E3 DNA regions. The preferred packaging cell line derived from human embryonic kidney cells (293 cell line) contains the adenovirus E1 and minimum essential ORF6 region of Ad5 E4 gene integrated into the 293 cell genome. In a particular embodiment, the packaging cell line is identified herein as 293-ORF6 and deposited on October 25, 1995 with the American Type Culture Collection (ATCC), 12301 Parklawn Drive, Rockville, Maryland, under the Budapest Treaty, and has there been designated ATCC # 11990.

Another object of the present invention is to provide a novel packaging cell line which complements functions of the E1, E2A and optionally the E3 gene regions of a defective adenovirus deleted of the E1, E2A and optionally E3 regions, thereby allowing the production of the above described second generation recombinant adenoviral vectors deficient of the E1, E2A and optionally, the E3 DNA regions. The preferred packaging cell line derived from human embryonic kidney cells (293 cell line) contains the adenovirus E1 and E2A gene regions integrated into the 293 cell genome.

Another, object of the present invention is to provide a novel packaging cell line which complements functions of the E1, E2A, E4 and optionally the E3 gene regions of a defective adenovirus deleted of the E1, E2A, E4 and optionally E3 regions, thereby allowing the production of the above described second and third generation recombinant adenoviral vectors deficient of the E1, E2A, E4 and optionally, the E3 DNA regions. The preferred packaging cell line derived from human embryonic kidney cells (293 cell line) contains the adenovirus E1, E2A and E4 gene regions integrated into the 293 cell genome.

Another object of the present invention is to provide a plasmid used to introduce the E4 region into the 293 cells. The bacterial plasmid comprises the adenovirus E4 region devoid of the E4 promoter and substituted with a cellular inducible hormone gene promoter that is regulated by a CRE binding protein such as α-inhibin, β-inhibin, α-gonadotrophin, cytochrome c, cytochome c oxidase complex (subunit IV) and glucagon. The E4 gene region is operably linked to the CREB promoter in the plasmid provided above. In a particular embodiment, the plasmid comprises the adenovirus described above and a mouse alpha (α)-inhibin promoter which is identified as pIK6.1 MIP(α)-E4 and deposited at the ATCC on August 30, 1994, under the Budapest Treaty, and has there been designated ATCC #75879.

Yet another object of the present invention is to provide a plasmid that introduces the minimal essential E4 gene region, open reading frame 6 (ORF6) region, into the 293 cells. The bacterial plasmid comprises the adenovirus ORF6 fragment of the E4 gene region devoid of the E4 promoter and substituted with a cellular inducible hormone gene. promoter that is regulated by a CRE binding protein such as α-inhibin, β-inhibin, α-gonadotrophin, cytochrome c, cytochome c oxidase complex (subunit IV) or glucagon. The ORF6 gene fragment is operably linked to the CREB promoter in the plasmid provided above. In a particular embodiment, the plasmid comprises the adenovirus ORF6 fragment and a mouse α-inhibin promoter which is identified as pIK6.1 MIP(α)-E4ORF6 and deposited at the ATCC on October 25, 1995 under the Budapest Treaty, and has there been designated ATCC # 97325.

Yet another object of the present invention is to provide a plasmid that introduces the adenovirus 5 E2A gene that encodes the adenovirus DNA binding protein (DBP) into the 293 cells. The bacterial plasmid comprises the adenovirus E2A gene region devoid of the E2A promoter and substituted with a cellular inducible, hormone gene promoter that is regulated by a CRE binding protein such as α-inhibin, β-inhibin, α-gonadotrophin, cytochrome c, cytochome c oxidase complex (subunit IV) or glucagon. The E2A gene fragment is operably linked to the CREB promoter in the plasmid provided above. In a particular embodiment, the plasmid comprises the adenovirus E2A gene and a mouse α-inhibin promoter which is identified as pIK6.1 MIP(α) - E2A and deposited at the ATCC on October 25, 1995 under the Budapest Treaty, and has there been designated ATCC # 97325.

### BRIEF DESCRIPTION OF THE DRAWINGS

Figure 1 depicts the construction of the pIK.6.1 MIP(α)-E4 plasmid, as described in Example 1, *infra*.
Figure 2 depicts the construction of the ADV-β-gal plasmid, as described in Example 1, *infra.*

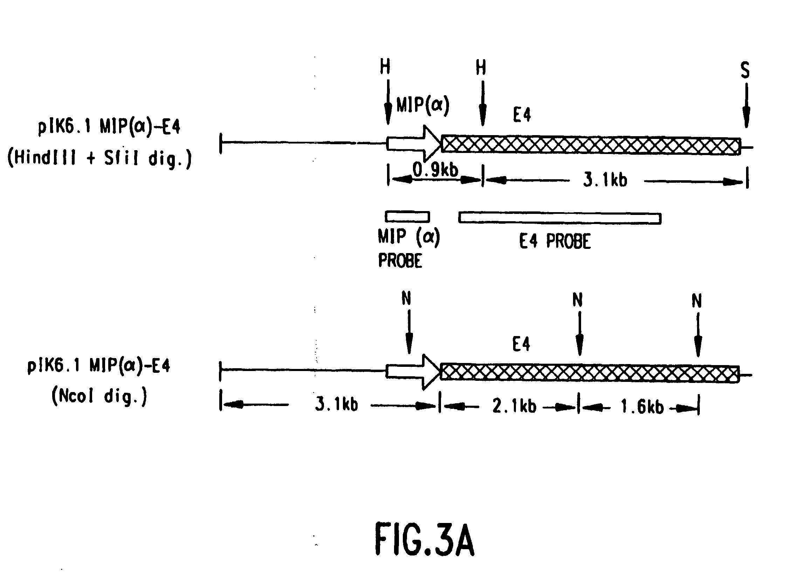

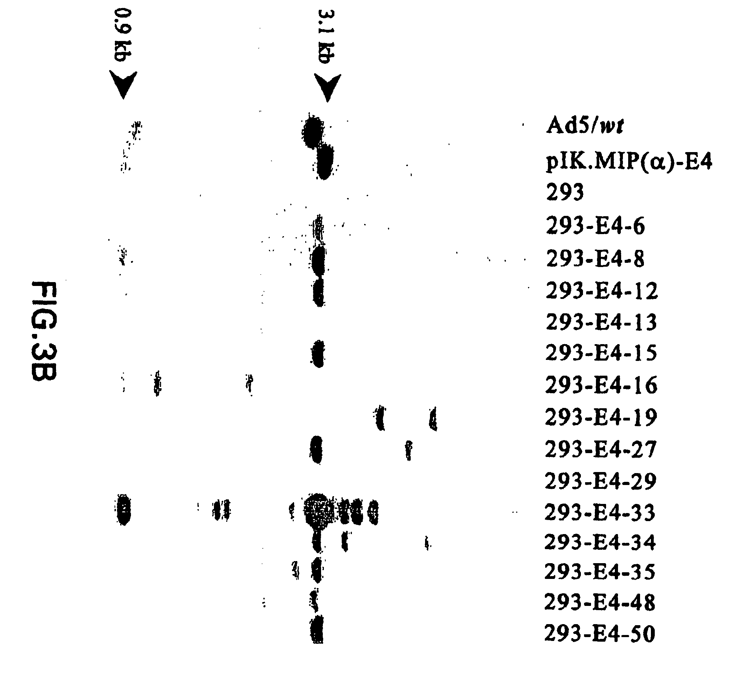

Figure 3 (A)-(E) are illustrations and the Southern analysis of 293-E4 cell lines as described in Example 3, infra. (A) The restriction patterns of the introduced MIP(α)-E4 and the probes used in Southern blots are depicted in this illustration. The solid arrow represents the mouse α-inhibin promoter region. The open box represents the full length of the E4 region. The mouse inhibin probe is the 283 bp PCR product described in Example 1. The E4 probe is the Sma I H fragment (m.u. 92 to 98.4). Restriction enzyme sites are abbreviated as follows: H, Hind III; S, Sfi I; N, Nco I. (B) DNA was digested with Hind III and Sfi I and hybridized to the E4 probe. (C) DNA was digested with Nco I and hybridised to the E4 probe. (D) The E4 probe was stripped from Hind III and Sfi I digestion blot and the DNA was reprobed with the inhibin promoter probe. (E) The inhibin probe was washed off from the Hind III and Sfi I digestion blot and DNA was reprobed with the E1 probe which is a Hind III E fragment from m.u. 7.7 to m.u. 17.1.

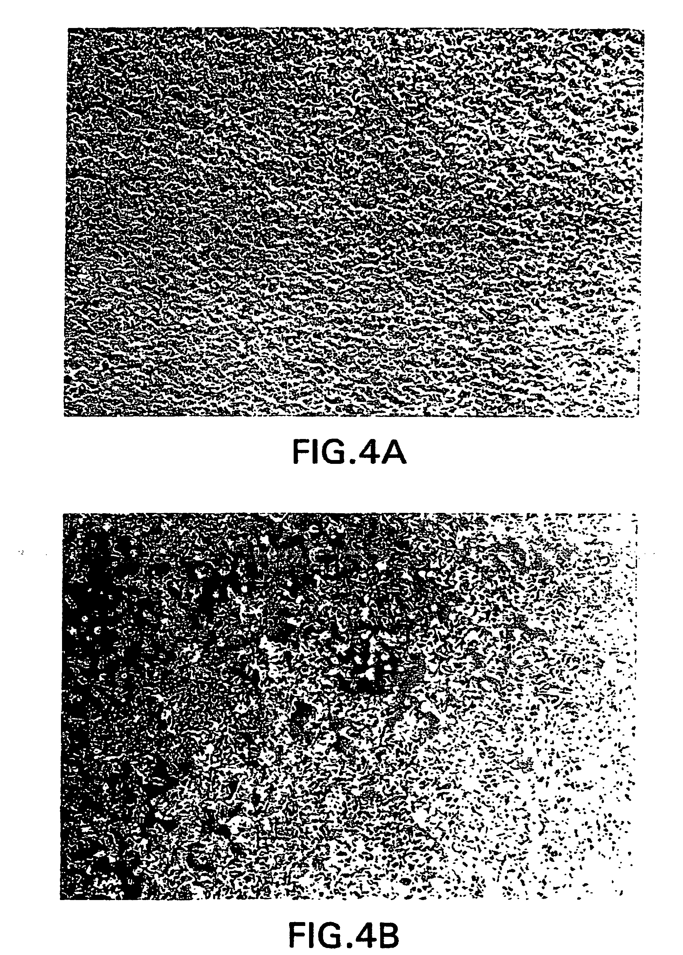

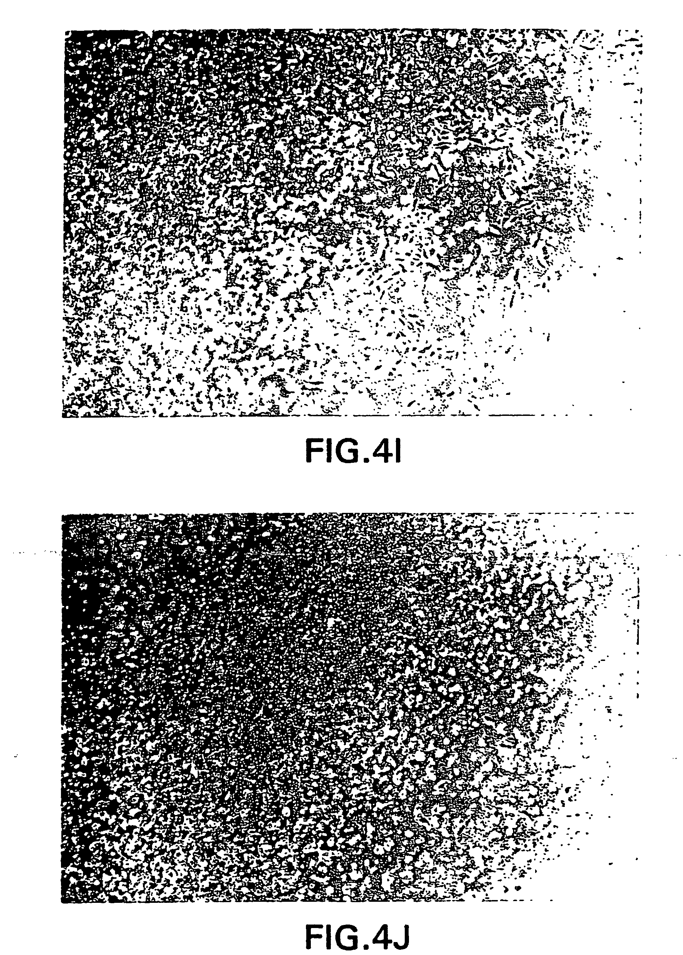

Figure 4 A-J are photographs showing the cytopathic effect of H5d11014 on W162, 293 and 293-E4 cell lines in the presence or absence of cAMP, as described in Example 10, *infra*. Parental 293 cells are represented in panels A-D; 293-E4 cells are represented in panels E-G and W162 cells are represented in panels H-J. The cells without infection are shown in panels A, E and H. The cells infected with H5dl1014 without an addition of cAMP are shown in panels C, F and I and the cells infected with H5dl1014 with an addition of 1mM cAMP are shown in panels D,G and J. Panel B represents 293 cells with a mock infection and an addition of 1mM of cAMP.
Figure 5 depicts the construction and the structure of recombinant viruses Ad5/ΔE1(β-gal)ΔE4 and Ad5/ΔEa(β-gal)ΔE3, as described in Example 5, *infra.*
Figure 6 illustrates the restriction enzyme analysis of recombinant viruses, as described in Example 5, *infra*.
Figure 7 represents the Northern analysis of transcripts in Hela cells infected with recombinant adenovirus vectors. Total RNA was isolated at 4, 24 and 48 hr post-infection. Panel A is the transcripts identified by hybridizing to a ³²P-labeled the β-gal DNA probe. Panel B is the transcripts hybridized with the Ad5 E4 probe. Panel C is the transcripts detected by hybridizing to the Ad5 L3 region DNA probe. Panel D is the transcripts probed with the radioactive labeled L5 region PCR product.
Figure 8 represents the ethidium bromide stained agarose gel of the RT-PCR products as described in Example 15, *infra*.
Figure 9 represents a Southern blot analysis of the L3 reverse transcription polymerase chain reaction (RT-PCR) products. The RT products from the +RT reaction mixtures were run on the agarose gel, transferred to nylon membrane and then probed with the end labeled oligomer hybridizing to the internal sequence of the L3 RT-PCR products.
Figure 10 illustrates the construction of the ORF-6 E4 plasmid as described in Example 18, *infra.*

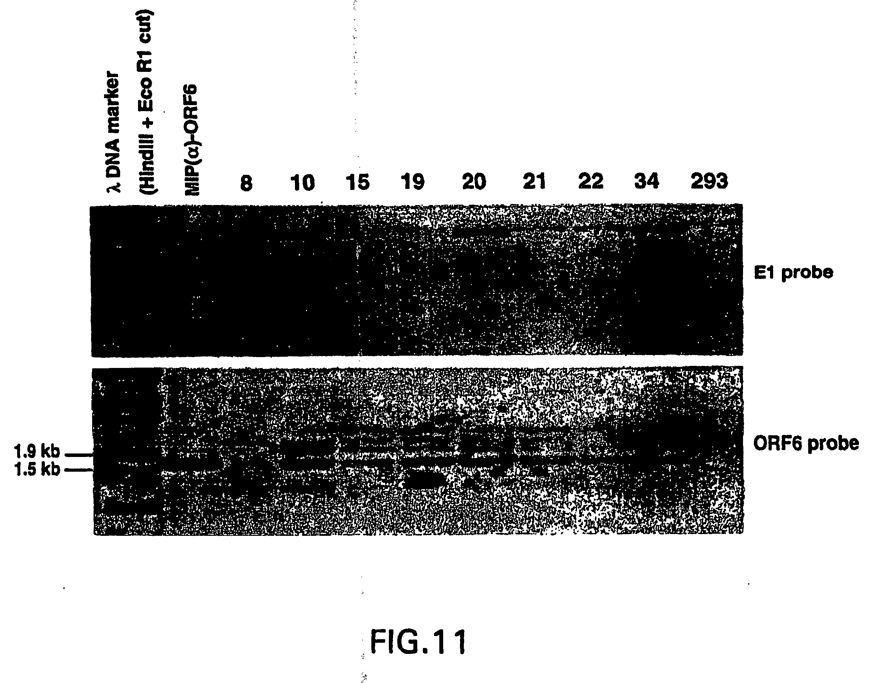

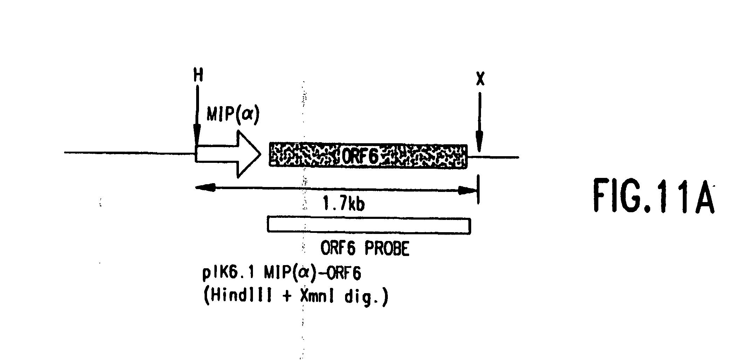

Figure 11 (A) is a diagramatic restriction pattern of pIK6.1MIP(α)-ORF6 plasmid. The plasmid pIK6.1MIP(α)-ORF6 contains 910 bp PCR product of adenovirus 5 E4-ORF6 coding sequence from nuleotide sequence 1876 to 2756 from right end of the viral genome which is under the control of the mouse α inhibin promoter. The open arrow represents the mouse α inhibin promoter region. The cross-hatching box represents the ORF6 coding region. The ORF6 probe is the PCR product. Restriction enzyme sites are abbreviated as follows: H, Hind III; X, XmnI. Figure 11 (B) represents a Southern blot of 293-ORF6 cell lines probed with the ORF6 probe (lower photograph). The same blot was rehybridized with the E1 probe (top photograph) which is a Hind III E fragment from m.u. 7.7 to m.u. 17.1.

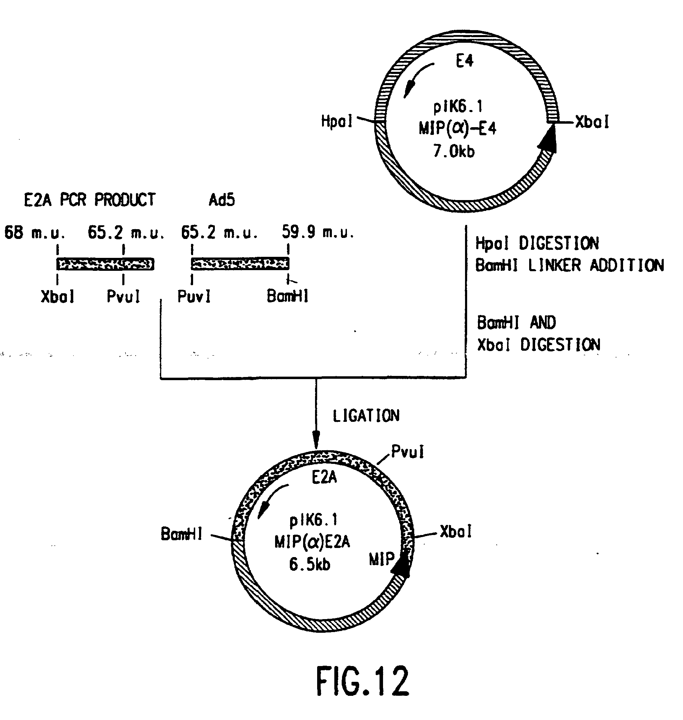

Figure 12 illustrates the construction of the pIK6.1MIP(α)-E2A plasmid.
Figure 13 depicts the construction of the plasmid comprising DNA sequences that transcribe the virus-associated RNA gene region.

### DETAILED DESCRIPTION OF THE INVENTION

One strategy designed to circumvent the problems associated with current early region-deleted adenoviral vectors is to introduce a second essential gene region deletion into the adenoviral vector. Several adenovirus early gene region transformed cell lines which support the growth of E1, E2A or E4 mutant virus growth, respectively, have been established [Grahan, et al, *J. Gen Virol*. **36:** 59-72 (1977), Weinberg, et al, *Proc. Natl. Acad*. *Sci. USA* **80:** 5383-5386 (1983) and Brough, et al, *Virology* **190:** 624-634 (1992)]. However, no cell line offers the functions of two gene regions simultaneously and at permissive temperatures. Establishing such a cell line which possesses the capability to complement the E1 and a second essential gene region function in trans (eg., E4), and the capacity to function as a packaging cell line for the propagation of recombinant viral vectors containing such double (or possibly triple or quadruple) deletions, may eliminate the drawbacks of the first generation adenoviral vectors currently available.

Studies of the adenovirus early region (ER) gene functions have shown that the deletion of the E4 region results in a failure to accumulate viral late transcripts; a reduction in viral late protein synthesis; a defective viral particle assembly and a failure to inhibit host protein synthesis at the late infection stage [Sandler, et al, *J. Viro*l.**63**: 624-630 (1989), Bridge & Ketner, *Virology* **174:** 345-353 (1990), Ross & Ziff, *J. Virol*. **66:** 3110-3117 (1992), Bridge, et al, *Virology* **193:** 794-801 (1993), and Bett, et al, *J. Virol*. **67:** 5911-5921 (1993)]. Dual removal of the E1 and E4 gene regions from the recombinant adenovirus vectors may therefore dramatically minimize or eliminate the pathogenic effects of direct cytotoxicity to the targeted cells and inflammatory responses in the human body. The E4 deletion in a second generation recombinant adenoviral vector would provide the additional benefit of increasing the capacity of this vector system to accommodate human gene inserts as large as 10 kb.

In one aspect of the present invention, the successful establishment of a novel packaging cell line which supports the growth of both the E1 and E4 deletions in E1 and E4 deficient adenoviruses has been demonstrated. Since one of the E4 gene products [294R protein of open reading frame (ORF) 6] in association with the E1b gene product (496R protein) has a function of inhibiting cellular mRNA transport resulting in the cessation of cellular protein synthesis (Bridge & Ketner, 1990), the overexpression of the E4 gene region would be expected to ultimately result in cell death. A major obstacle to the introduction of the E4 gene region into 293 cells has been overcome, i.e., the trans activation of the E1a gene product in the parental 293 cells which causes the overexpression of the E4 genes which would otherwise result in cell death. In the present invention, the E4 promoter is replaced with a cellular inducible hormone gene promoter, namely, a gene that is regulated by a nuclear, factor called CRE binding protein (CREB). Particularly, the promoter that replaces the E4 promoter is chosen from the CREB regulated gene family such as α-inhibin, beta (β)-inhibin, α-gonadotropin, cytochrome c, cytochrome c oxidase complex (subunit IV), glucagon, etc. listed in Table I on page 15695 in Kim, et al, *J. Biol Chem*., **268:** 15689-15695 (1993). In a preferred embodiment, the CREB regulated gene promoter is a mammalian α-inhibin, most preferably, mouse α-inhibin. In this instance, a 165 base pair sequence of the mouse inhibin promoter region has been shown to drive the heterologous gene expression at a low basal level and increase the levels of heterologous gene expression in response to the induction of cAMP or adenylic cyclase activators [Su & Hsueg, *Biochem. and Blophys. Res. Common*. **186**, 293-300 (1992)]. An 8 bp palindromic sequence called cAMP response element (CRE) is responsible for this inductory effect and has been identified within the inhibin promoter region. In fact, all adenovirus early gene promoters contain the CRE-like element which renders these early genes responsive to the induction of cAMP [Jones, et al, *Genes Dev.* **2:** 267-281 (1988)]. It is clear that E1a trans activation and the cAMP enhancement act on adenovirus early genes via independent mechanisms [Leza & Hearing, *J. Virol*. **63:** 3057-3064 (1989) and Lee, et al, *Mol. Cell. Biol*. **9:** 4390-4397 (1989)]. The replacement of the E4 promoter with the mouse α-inhibin promoter uncouples the E1a trans-activation from the cAMP induction on the E4 gene. In the present invention, a full length sequence of the E4 region is introduced into the 293 cells whereby the cAMP induction is still effective in inducing E4 gene expression in the transformed cells in a controlled manner. It should also be noted that this novel 293-E4 packaging cell line may also rescue (supports the growth of) adenoviruses containing the E3 deletion in addition to the E1 and E4 deletions because the deletion of the E3 region will not affect the viability of the virus.

In accordance with the present invention, bacterial plasmids are prepared using standard cloning procedures and starting materials described in Finer, et al 1994 and Finer et al WO 94/29438. The parental plasmid pIK6.1 MMSV-E4 (ΔE4pro) is derived from pIK6.1.MMSVNhe (Finer et al WO 94/29438) and contains the full length sequence of the adenoviral E4 region except for the absence of the E4 promoter which is substituted with the MMSV promoter. Using cloning techniques well known in the art, the MMSV promoter is replaced with one of the CREB regulated promoters described above. In a preferred embodiment, the promoter that is operably linked to the adenoviral E4 promoterless gene region is mammalian alpha inhibin, most preferably, derived from the mouse. The resulting preferred plasmid is designated pIK6.1 MIP(α)-E4 and deposited at the ATCC, Rockville, MD under the terms of the Budapest Treaty as ATCC #75879. The plasmids containing the CREB regulated promoters operably linked to the adenoviral E4 gene fragment, ORF6 or adenoviral E2A gene was constructed using the above pIK6.1 MIP(α)-E4 plasmid as the starting material. The promoterless E4 region was replaced with a PCR product of the ORF6 fragment of the E4 gene or E2A gene region to construct the pIK6.1 MIP(α)-ORF6 and pIK6.1 MIP(α)-E2A plasmids that are operably linked to α-inhibin promoter. Plasmids were deposited at the ATCC, Rockville, MD having the chararacteristic features of the above described ORF6 and E2A plasmids operably linked to mouse (α)-inhibin having ATCC #97325 and ATCC # respectively. In accordance to the present invention, one may use any of the CREB regulated promoters in substitution of the inhibin promoter and achieve similar results when the plasmid is transfected into the packaging cell lines described below.

The novel 293-E4 packaging cell lines were stably transformed by the E4 region and displayed the same morphology and the growth rate as parental 293 cells. This indicates that the low level of E4 gene expression under the control of the mouse α-inhibin promoter does not cause extensive inhibition of host cell protein synthesis. The mutant adenovirus, H5d11014 [Bridge, et al, *Viroiogy* **193:** 794-801 (1993)], was used to examine the complementing activity of the above described 293-E4 packaging cell line because it carries lethal deletions in the E4 region and can only grow in W162 cells (Bridge, & Ketner, 1989). The W162 cell line is a Vero (monkey kidney) cell line transformed by adenovirus E4 DNA and complements the growth of E4 deletion adenoviruses. The H5d11014 virus has been shown to produce markedly reduced levels of DNA and failed to synthesize late protein due to an intact ORF 4 [Bridge, et al, (1993)] in its mostly deleted E4 region. Cell lines were found that produced the H5dl1014 virus at comparable titers to that produced in W162 cells (See Table IV, Groups 1 and 2 in Example 11, *infra*).

In another embodiment, the present invention relates to a method of producing replication-defective adenoviruses said method comprising (a) transfecting packaging cell lines of the present invention. with a recombinant adenoviral vector said adenovival vector comprising either (i) a transgene and a lethal deletion or mutation in the E4 early gene region and a lethal deletion or mutation in the EI early gene region; or (ii) a transgene, a lethal deletion or mutation in the E2A early, gene region, and a lethal deletion or mutation in the E4 early gene region and the E1 early gene region; and (b) rescuing replication-defective adenovirus produced. As described herein, the term "recombinant adenovirus" or "recombinant adeno-associated virus" (also known as recombinant viral vectors in the art) refers to a virus wherein the genome contains deletions, insertions and/or substitutions of one or more nucleotides, and the virus further carries. a transgene.

In one particular aspect of this embodiment, the novel 293-E4 packaging cell lines described above are used to generate a second generation of recombinant virus called Ad5/ΔE1(β-gal)ΔE4. Although the 293-E4 packaging cell line contains the adenoviral serotype 5 E1 and E4 gene regions, other serotypes of mutant and recombinant adenoviruses, for example, serotype 2, 7 and 12, may be rescued due to the high degree of structural and functional homology among the adenoviral serotypes. Moreover, mutant and recombinant adenoviruses from serotypes other than serotype 5 may be rescued from the other novel adenoviral packaging cell lines of the present invention described *infra.*

*In vitro* studies demonstrate that the infection of the novel recombinant adenovirus vectors of the present invention in non-permissive human cells show no cytopathic effects and the efficiency of the transgene expression is at levels comparable to conventional E1-deleted viruses. It is expected that the host immune responses and inflammatory reactions at the sites infected with novel second generation recombinant adenoviruses of the present invention will be reduced compared to the first generation recombinant adenoviruses currently available. The establishment of the dual complementing packaging cell line of the present invention marks a significant event in the evolution of safer and more effective gene transfer adenoviral vectors. The method used in the construction of the 293-E4 cell lines of the present invention is of general utility in the production of other packaging cell lines which contain additional adenoviral regions which complement further deletions of the adenoviral vectors of the present invention or in the construction of other viral vectors.

Thus, in another embodiment, the present invention relates to novel adenoviral packaging cell lines that can rescue deletions in addition to E1, E4 and optionally E3 by the methods described above. In this example, an adenoviral vector packaging cell line which can rescue the E2A mutation or deletion, in addition to the E1, E3 and E4 deletions, was constructed starting with the novel packaging cell line described above, namely the 293-E4 packaging cell line. The E2A gene product is a regulatory protein, specifically, a DNA binding protein. This gene may be introduced into the 293-E4 packaging cell line by placing the E2A gene under the control of an inducible promoter operably linked to the E2A gene in a similar manner as described above. The inducible promoter may be selected from the same family of CREB regulated genes described above used to replace the E2 gene promoter.

In yet another embodiment, the present invention relates to an adenoviral vector packaging cell line that may rescue the adenovirus recombinant virus containing the minimum essential cis-elements (inverted terminal repeats (ITRs) and packaging signal sequence) [Hering, et al, *Virol.* **61**: 2555-2558 (1987)] and protein IX sequence [Ghosh-Choudury, et al, *EMBO J.* **6**: 1733-1739 (1987)] only. This cell line may be established by introducing the adenovirus DNA sequence from around m.u. 11.2 to approximately 99 into the novel 293-E4 cell line described above. A plasmid carrying the above adenovirus DNA sequence may be constructed and transfected into the 293 cells. This DNA sequence represents the sequence from after E1b gene to the 3' end of the viral structural gene [Sanbrook, et al, *Cold Spring Harbor Symp. Quant. Biol*. **39:** 615-632 (1974); Ziff & Evans, *Cell* **15**: 1463-1476 (1978)]. The introduced adenovirus sequence contains viral structural genes and almost the entire functional gene regions except E1a and E1b. Because the constitutive expression or overexpression of viral gene products are very toxic to the cells, the introduced adenoviral DNA may be manipulated to replace adenoviral native promoters with heterologous promoters. For example, the early gene regions which encode viral regulatory proteins may be placed under the control of the CREB regulated promoters, which have about 2 to 10 fold induction efficiency. In the case of the gene region that encodes viral structural proteins, the native major late promoter may be replaced by a tightly controlled exogenous promoter such as the tetracycline-responsive promoter which has an induction level up to about 10⁵ fold in the presence of tetracycline [Manfred & Hermann, *PNAS* **89**: 5547-5551 (1992)].

In another embodiment, the present invention relates to novel adenoviral-associated (AAV) packaging cell lines prepared in the following manner. The novel complementing cell. line contains the E1a, E1b, E2A, and E4 gene regions and the DNA sequence encoding virus-associated RNA. This cell line may be constructed by introducing the adenovirus DNA sequence encoding the virus-associated RNA (around 600 NTs from m.u. 28-30) [Mathews, *Cell* **6**: 223-229 (1975) and Petterson & Philipson, *Cell* **6**: 1-4 (1975)] into the novel 293-E4 packaging cell line constructed above that rescues the E1 and E4 deletions, the E2A mutation of adenovirus and optionally E3. The wild type AAV produced from this packaging cell line will be free of helper adenovirus. The recombinant adeno-associated virus or mutant AAV will only contain the minimal essential cis-elements and will be generated by co-transfecting a non-packaging complementing AAV plasmid which is defective for packaging but supplies the wild type AAV gene products [Samulski et al, *J. Virol*. **61:** 3096-3101 (1987)]. Moreover, the recombinant adeno-associated viral vectors or mutant AAV rescued from this cell line will be free of helper viruses, i.e., adenoviruses.

In another embodiment, the present invention relates to yet another novel AAV packaging cell line constructed by starting with the AAV packaging cell line described above. This packaging cell line contains the E1a, E1b, E2A and E4 gene regions, the DNA encoding virus-associated RNA and additionally, the AAV virus replication (rep) gene regions. The rep gene region encodes at least four replication (Rep) proteins that are essential for AAV DNA replication and trans-regulation of AAV gene expression [(for review, see Bervis & Bolienzsky, *Adv. Virus Res.* **32:** 243-306 (1987)]. It is constructed by introducing the AAV rep gene region into the AAV packaging line described above that already contains the E1, E2A, E4 gene regions and DNA sequences encoding the virus-associated RNA in the manner that replaces the P5 promoter [(Yang, et al, *J. Virol*. **68:** 4847-4856 (1994)] with an inducible promoter chosen from the CREB regulated gene family described previously. The novel AAV virus and its recombinant virus rescued from the cell line will be free of helper viruses (adenoviruses) and is Rep- [Muzyczka, *Curr. Top. Microbiol. Immunol.* **158:** 97-129 (1992)].

In another embodiment, the present invention relates to another novel AAV packaging cell line constructed by starting with the AAV packaging cell line described in the previous paragraph. This packaging cell line contains the E1a, E1b, E2A, E4 gene regions, the DNA encoding the virus-associated RNA, the AAV virus replication (rep) gene region, and additionally the AAV cap gene region. The cap gene region encodes a family of capsid proteins, i.e., VP1, VP2 and VP3 [Janik, et al, *J*. *Virol*. **52:** 591-597 (1984)]. The synthesis of all three mRNAs are started from a single promoter called P40 [Janik, et al, (1984)]. This gene region will be introduced into the AAV packaging cell line described above by replacing the P40 promoter with an inducible promoter selected from either the CREB regulated promoters or the tetracycline responsive promoter. The novel AAV virus and its recombinant virus rescued from the cell line will be free of helper viruses (adenoviruses) and only contain the minimal essential cis-elements [Muzyczka, *Curr. Top. Microbiol. Immunol*. **158:** 97-129 (1992)].

In yet another embodiment, the present invention provides a particular second generation packaging cell line for the. propagation of both E1 and E4 deleted vectors and viruses. This line has been establisheed by the introduction of the minimum essential E4 gene region, open reading frame 6 (ORF6) region, driven by the mouse α-inhibin promoter and provides the same function as the cell line designated 293-E4 described above. It is expected that the use of this packaging cell line for the production of E1, E4 and double-deleted recombinant adenoviral vectors will eliminate the problem of any possible homologous recombination event in the E4 region. Thus, the expansion and passage of purified stocks of E1/E4 deleted recombinant adenovirus, for example, should be absolutely free of any contamination by replication-competent adenovirus (RCA) particles. The strategy of creating this safer duel packaging cell line was to introduce a 910-bp DNA fragment which only comprises the ORF6 coding region (Ad5 nuleotides 1876-2756 from right end of the genome) into 293 cells instead of a full length of the E4 gene region. There are many existing E4 deletion mutant viruses. Those displaying a severe defective phenotype are all with E4 deletions extending substantially beyond the region of the ORF6. For example, some of these deletions are as follows: NTs 575 to 2845 as the boundary of the two deletions within the E4 region of the H5dl1014; same endpoints of the deletion in the H5dl366; from 932/937 to 2942/2946 within tandem repititions sequences of the H2dl808; and from 981 to 2845 in the H5dl1004. Due to lack of overlapping sequences between the integrated ORF6 DNA fragment and a recombinant adenovirus vector which carries a large E4 region deletion, the repairment of the E4 deletion through homologous recombination becomes essentially zero.

Previous reports have indicated that either the ORF3 or ORF6 gene fragment alone is sufficient to provide the E4 function necessary for normal adenovirus life cycle. It is believed that the ORF3 and ORF6 gene segments have redundant functions involved in viral DNA replication, late viral mRNAs transport and accumulation and host cell shut off. Although other ORFs of the E4 region have important regulatory roles in the multiplication of the virus, they are dispensible. To confirm that the provided 293-ORF6 cell lines of the present invention not only contain intact E1 and ORF6 DNA sequences but also possess the complementing activities of E1 and E4 functions, an E1-deleted mutant virus, an E4-deleted mutant virus as well as the E1/E4-deleted recombinant virus were used to infect the 293-ORF6 cell lines. The titers of these viruses measured on individual 293-ORF6 monolayers were shown to be compatible to the titers measured on each virus' permissive cell line. Therefore, the 293-E1/ORF6 packaging cell line of the present invention not only meets the safety requirment for use in human subjects but also efficiently produces E1 or E4 deletion mutant viruses, and double deleted E1/E4 viruses and vectors. This cell line has been deposited at the ATCC in Rockville, MD on October 25, 1995 and designated ATCC # 11990.

In another embodiment, the present invention provides for a 293-E2A packaging cell line that complements both the E1 and E2A gene functions in trans simultaneously. The human adenovirus 72 Kd DNA-binding protein (DBP) is important in the infectious cycle of the virus. At non-permissive temperature, the ts mutations within the DBP coding region (E2A region) inhibit viral DNA replication [Friefeld, et al, *Virology* **124**: 380-389 (1983)] and fail to regulate early gene expression in late stage of viral life cycle [Carter, et al, *J. Virol*. **25**: 664-674 (1978)]. Although the generation of E1-deleted, E2A-mutated (ts mutation) adenovirus vector does not require a special packaging cell line, the ts DBP mutation may not give rise to a full inactive gene product in the temperature permissive in vivo condition [Engelhardt, et al, *Proc. Natl. Acad. Sci. USA,* **91**: 6196-6200 (1994)]. A deletion within the indispensible region of the E2A gene (the gene region encoding the carboxyl-terminal protion of the DBP) is lethal to the adenovirus [Vos, et al, *Virology* **172**: 634-642 (1989)] both in vitro and in vivo. To generate a recombinant vector containing both E1 and E2A gene region deletions, establishment of a complementation cell line becomes absolutely necessary. The present invention provides an adenoviral packaging system where recombinant adenoviral vectors and mutant adenoviruses are created. It is expected that the combination of the E1 deletion and E2A deletion of a recombinant adenovirus vector will result in complete replication-incompetence and safer to use in humans.

In yet another embodiment, the present invention further provides for a triple packaging cell line that is able to complement the functions of the adenoviral E1, E2A and E4 gene regions in trans simultaneously. The recombinant adenovirus vector generated from this packaging cell line harbors three early gene region deletions which renders the packaged adenoviral vector absolutely safe for all human applications with the added benefit of extensive capacity for larger size transgene insertions.

The present invention further provides the production of replication-defective adenoviruses containing a transgene which will be expressed in the target cells. The replication-defective adenovirus are prepared using the packaging cell lines described above which comprise one or more distinct nucleotide sequences capable of complementing the part of the adenovirus or AAV genome that is essential for the virus' replication and which is not present in the replication-defective adenovirus. Replication-defective adenovirus will no longer contain genes required for the virus replication in infected target cells. More particularly, the replication-defective adenovirus will only contain the minimum essential cis-elements (i.e., ITRs and packaging signal sequence) and protein IX sequence, and be free of the E1 (specifically, E1a and E1b) and E4 regions, and may additionally be free of E3 and E2A regions and the viral structural genes.

The replication-defective adenovirus is also characterized in that it is capable of directing the expression and the production of the selected transgene product(s) in the targeted cells. Thus, the replication-detective adenovirus comprise at least all of the sequences of the adenoviral DNA sequence essential for encapsidation and the physical structures for infection of the targeted cells and a selected transgene which will be expressed in the targeted cells.

The transgene may encode a cytokine gene, suicide gene, tumor suppressor gene, protective gene viral protein, or a combination thereof chosen from the list provided in Table II. If a cytokine gene is selected, the expression of the gene in a targeted cell may provide a treatment to malignancies by stimulating cellular immune responses which result in suppression of tumor growth and/or killing of tumor cells. If a suicide gene is chosen, the gene when expressed in the tumor cell will enable the tumor cell to be destroyed in the presence of specific drugs. For example, the thymidine kinase gene when expressed in tumor cells will enable the tumor to be destroyed in the presence of gancyclovir.

The following examples are presented to illustrate the present invention and are not intended in any way to otherwise limit the scope of this invention.

### EXAMPLES

### Example 1

### Construction of plasmids

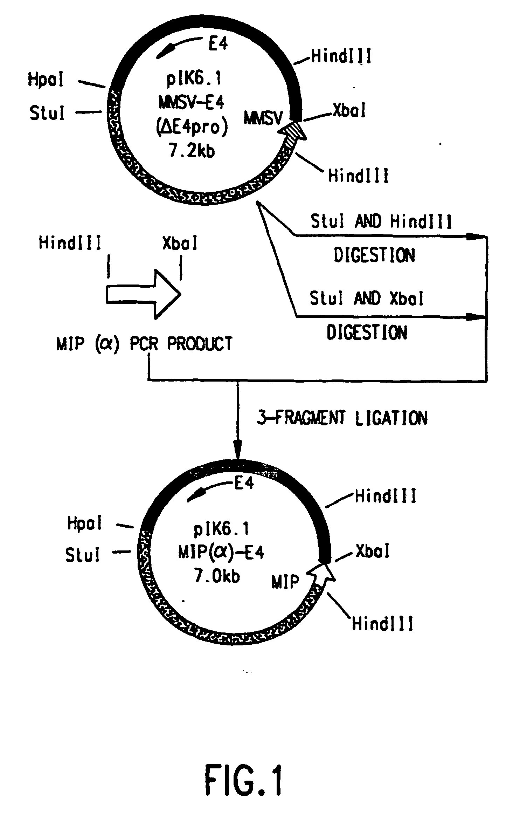

This example describes the construction of the plasmids used to introduce the E4 gene region into the 293 cells. The constructed plasmids are diagrammatically represented in Fig. 1. The parental plasmid pIK6.1 MMSV-E4 (ΔE4 pro.) derived from the pIK6.1 MMSV enpoNhe(Hpa) [Finer, et al, *Blood* **83**: 43-50, (1994)] contains the promoterless E4 region from 15 bp upstream of the transcription start site to 810 bp downstream of the E4 polyadenylation site. The E4 gene is linked to the Moloney murine sarcoma virus U3 fragment. The pIK6.1. MIP(α)-E4 was constructed by ligation of a 238 bp fragment of the Hind III -XbaI PCR product of mouse alpha inhibin promoter [MIP(α)] (Su, & Hsueh, *Biochem. and Biophys. Res. Common.* **186:** 293-300, 1992) with the 2.9 kb XbaI-StuI fragment and the 3.9 kb Stu I-Hind III fragment of the PIK6.1 MMSV-E4 (E4 pro.). The primers used for PCR of the HIP (α) were 5'-gcgcaagcttcGGGAGTGGGAGATAAGGCTC-3' (SEQ ID NO:1) and 5'-ggcctctagaAGTTCACTTGCCCTGATGACA-3' (SEQ ID NO:2). The sequences containing either the Hind III site or Xba I site in lower case are present to facilitate cloning. The cloned α-inhibin promoter was sequenced to verify the accuracy of the sequence.

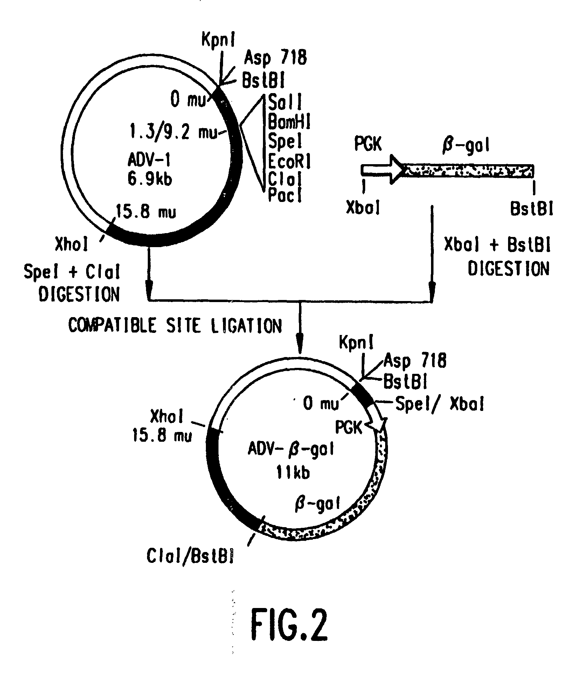

The plasmid ADV-β-gal used to generate recombinant adenoviruses was constructed as shown in Fig. 2. The starting plasmid ADV-1 contains the left end of adenovirus 5 Xho I C fragment (m.u. 0-15.8) with a deletion from nucleotides 469-3326 (m.u. 1.3-9.24) on the backbone of PCR II (In Vitrogen, San Diego, CA). A polylinker cassette was inserted into the deletion site. Several restriction sites at the left end of the adenovirus sequence can be conveniently used to linearize the plasmid. The resulting ADV-β-gal plasmid was constructed by insertion of a Bst BI-Xba I fragment of the *E. coli* β-galactosidase gene driven by the mouse pgk promoter into the ADV-1 compatible sites Spe I and Cla I in the E1 region and was later used to generate the recombinant virus.

### Example 2

### Transfection and selection of 293-E4 cell lines

This example describes the transfection and selection process employed to establish 293-E4 cell lines. The 293 cells, obtained from the American Type Culture Collection, ATCC #CRL 1573, were grown in Dulbecco's modified Eagle's medium (DMEM), 1g/L glucose (JRH Biosciences), 10% donor calf serum (Tissue Culture Biologics). Cells were seeded at 5x10⁵ per 10-cm plate 48 hours prior to the transfection experiment. Ten µg of pIK.MIP(α)-E4 and 1 µg of pGEM-pgkNeo.pgkpolyA containing the Neo^{r} gene were co-transfected into 293 cells by calcium phosphate co-precipitation [Wigler, et al, *Cell* **57**: 777-785 (1979)]. The transfected cells were split 1:20 in normal medium at 24 hours post-transfection. After the cells were attached to the plate, the medium was changed to selective medium containing 1 mg/ml G418 (Sigma, St Louis, MO). The cells were refed with fresh selective medium every 3 days for about 2-3 weeks. Isolated clones were picked, expanded and maintained in the selective medium for 5-6 passages. The established 293-E4 cell lines were routinely maintained in the normal medium.

### Example 3

### Southern transfers and hybridization

Genomic DNA from 293-E4 cell lines were digested with desired restriction enzymes and purified with phenol/chloroform. 10 µg of digested DNA were run on 0.8%-1% agarose gel and transferred to a nylon membrane (Zetabind, America Bioanalytical, Natick, MA). DNA from the 293-E4 cell lines were digested with restriction enzymes and analyzed. DNA from wild type adenovirus 5, pIK6.1 MIP(α)-E4 plasmid and parental 293 cells were also digested with the same enzymes and used as controls. Restriction fragments of the E4 region, α-inhibin promoter sequence, and the E1 region were detected by hybridization to the appropriate ³²P-labeled probes and subsequent autoradiography.

### Example 4

### Preparation of viral stocks

W162 cells were grown in DMEM, 4.5g/L glucose and 10% CS. The W162 cell line is a Vero monkey kidney cell line transformed by adenovirus E4 DNA and supports the growth of E4 deleted adenovirus mutants [Weinberg, & Ketner, *Proc*. *Natl. Acad. Sci. USA* **80:** 5383-5386 (1983)]. The H5dl1014 virus has been previously described in Bridge & Ketner, *J. Virol*. **63:** 631-638, (1989). This adenovirus 5 virus strain has two deletions within the E4 region and can only grow in W162 cells (Bridge, & Ketner 1989). Propagation and titration of H5dl1014 virus were done on W162 cells. For evaluation of the production of H5dl1014 virus from 293-E4 cell lines of the present invention, the W162, 293 and 293-E4 cell lines were counted and plated in the 6-well plate at 1 x 10⁵/well and infected with H5dl1014 at a multiplicity of infection (m.o.i.) of 50 plaque-forming units (p.f.u.) per cell. The viral stocks were prepared by harvesting the cells at 48 hr post-infection. The cells were precipitated and resuspended in 200 µl of serum free medium. The cell suspensions underwent 3 cycles of freeze and were thawed to release the viral particles from the cells. The cell debris was discarded by centrifugation. The titers of the virus produced from the infected cells were determined hy plaque formation on monolayers of W162 cells.

### Example 5

### Construction of recombinant viruses

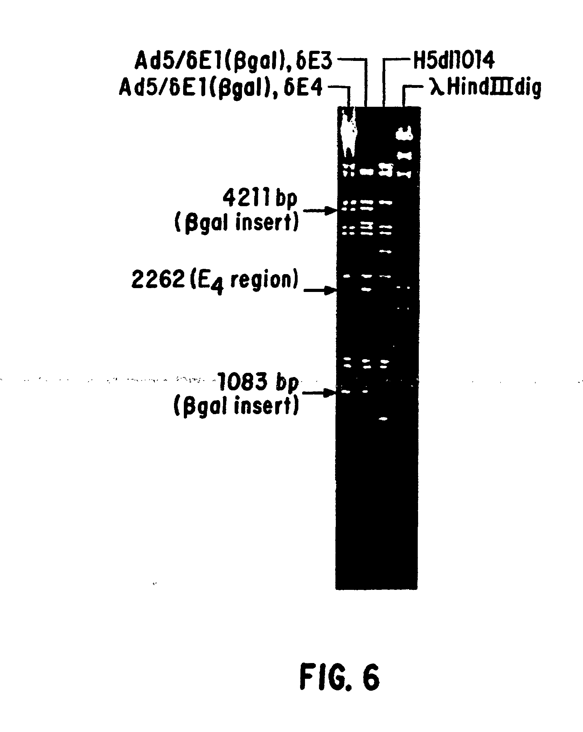

The 293 cell line and 293-E4 cell line were plated in 10-cm plate at 2.5 x 10⁶/plate 48 hours before the experiment. One hour prior to the co-transfection, cells were fed with 10 ml fresh medium. Ad5/ΔE1(β-gal)ΔE3 virus was made by co-transfection of 10 µg of ADV-β-gal linearized by Bst BI with 4 µg of H5d1327 (Thimmappaya, et al, *Cell* **31**: 543-551 1982) digested with Cla I. Ad5/ΔE1(β-gal)ΔE4 virus was generated by co-transfection of 10 µg of Bst BI linearized ADV-β-gal and 4 µg of Cla I digested H5dl1014 on 293-E4 cell lines by calcium phosphate precipitation technique. Twenty-four hours after co-transfection, the medium was removed and the monolayers of the culture were overlaid with 10 ml DMEM medium containing 20 mM MgCl₂, 5% of CS and 0.5% of noble agar (DIFCO Lab. Detroit, MI). The plaques were picked and resuspended in 100 µl of PBS. Diluted plaque samples were immediately subjected to 2 to 3 rounds of blue plaque purification. The blue plaque purification was carried out as a regular plaque assay except that the cultures were overlaid with a second layer of soft agar containing 1 mg/ml X-gal when plaques appeared. After incubation for 2 hours, plaques which contained the recombinant yirus carrying the β-galactosidase gene were stained blue. The purity of the recombinant virus was determined by no contamination of white plaques. The purified plaques were expanded and the DNA of the lysate was analyzed (Fig. 6) as previously described [Graham & Prevec (1992)]. Adenoviral DNA was digested with Sma I and fractionated on 0.8% agarose gel. DNA samples of H5dI1014 and the Ad5/ΔE1(β-gal)ΔE3 viruses were extracted from CsCl gradient purified viral stocks. DNA of the Ad5/ΔE1(β-gal)ΔE4 was extracted from the virus infected cells.

### Example 6

### Histochemical staining

Forty-eight hours following recombinant viral infection with Ad5/ΔE1(β-gal)ΔE3 virus (E1 and E3 deletion viruses) and Ad5/ΔE1(β-gal)ΔE4 virus (E1 and E4 deletion viruses) at 20 m.o.i. the monolayers of cells are washed once in PBS and fixed for 10 min. at room temperature with 0.5% glutaraldehyde (Sigma, St. Louis, MO) in PBS. The cells were washed three times with PBS containing 1 mM MgCl₂ and then stained with 5-bromo-4-chloro-3-indolyl-β, D-galactosidase (X-gal, Sigma) as previously described (Thimmappaya et al, 1982). The X-gal solution at 40 mg/ml in dimethylformamide was diluted to 1 mg/ml in KC solution (PBS containing 5 mM K₃Fe (CN)₆, 5 mM K₄Fe (CN)₆•3H₂O). After staining, for 2 - 4 hours the cells were washed with H₂O and inspected under a light microscope.

### Example 7

### β-galactosidase activity assay

Cells were infected with either Ad5/ΔE1(β-gal)ΔE3 virus and Ad5/ΔE1(β-gal)ΔE4 virus at 20 m.o.i. assayed for enzyme activity as described in MacGregor, et al, *Somatic Cell Mol. Genetic.* **13:** 253-264, (1987) with the following modifications. Cells in 6-well plate were washed with PBS twice and lysed in the well by addition of 200 µl of 2x Z buffer (lx Z buffer: 60 mH Na₂PO4•7H₂O, 40 mM NaH₂PO₄•H₂O, 10 mM KCl, 1 mM MgSO₄•7H₂O) and 200 µl of 0.2% Triton X-100. After incubation at room temperature for 5-10 min, 100 µl of each sample was transferred to the 96-well microtiter plate. After addition of 50 µl of 2-nitrophenyl-β-D-galactopyranoside (2mg/ml), the reaction was allowed to proceed for 5 min at room temperature and stopped by adding 50 µl of stop solution (1 M Na₂CO₃). Fluorescence was measured at 420 nm on a microtiter plate reader (Molecular Devices Co. Menlo Park, CA).

### Example 8

### Construction of 293-E4 cell lines

The purpose of introducing the Ad5 E4 gene region into 293 cells is that the derived cell line is able to package the recombinant adenoviruses containing two lethal deletions (E1 and E4). The plasmid, PIk.MIP(α)-E4 carries the full length region of the Ad5 E4 region from 15 bp upstream of transcription start site to 810 bp downstream of the polyadenylation site (Fig. 1). The E4 gene region (m.u. 88.9 - 98.8) was directly linked to 238 bps of the mouse α-inhibin promoter containing the first 159 bps of the promoter region and 5' untranslated region. This promoter sequence is required for basal expression (Su & Hseuh (1992)). Within this promoter region, there is a cyclic adenosine 3', 5'-monophosphate (cAMP) response element (CRE) which allows an increased level of gene expression induced by either cAMP or adenylic cyclase activator [Paei, et al, *Mol. Endocrinol*. **5**: 521-534 (1991)]. The pIK.MIP(α)-E4 was introduced into 293 cells together with the pGEM-pgkNeo.pghpolyA which bears a neomycin resistant gene by calcium phosphate precipitation at a molar ratio equivalent to 10:1. A total of 66 G418 resistant clones were picked for further analysis.

### Example 9

### Identification of E4 transfectants

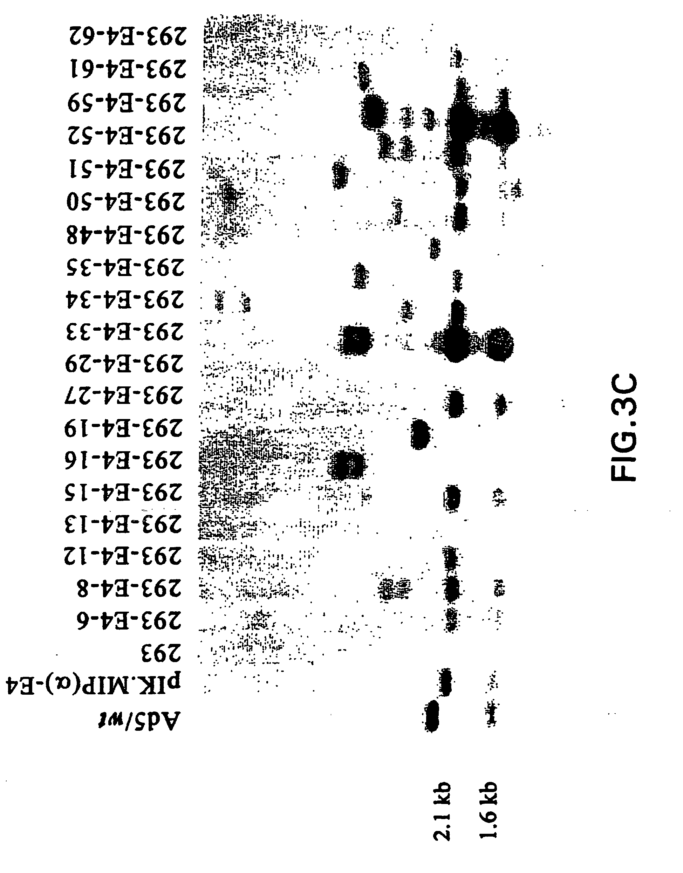

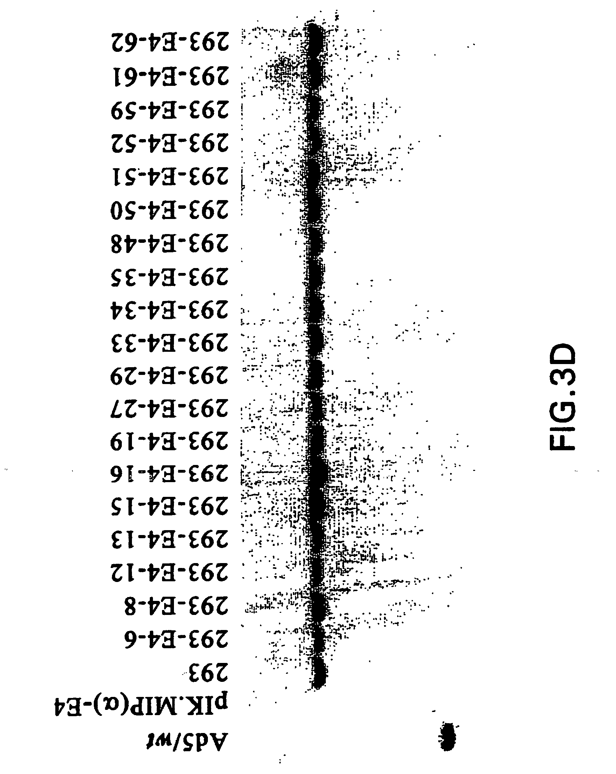

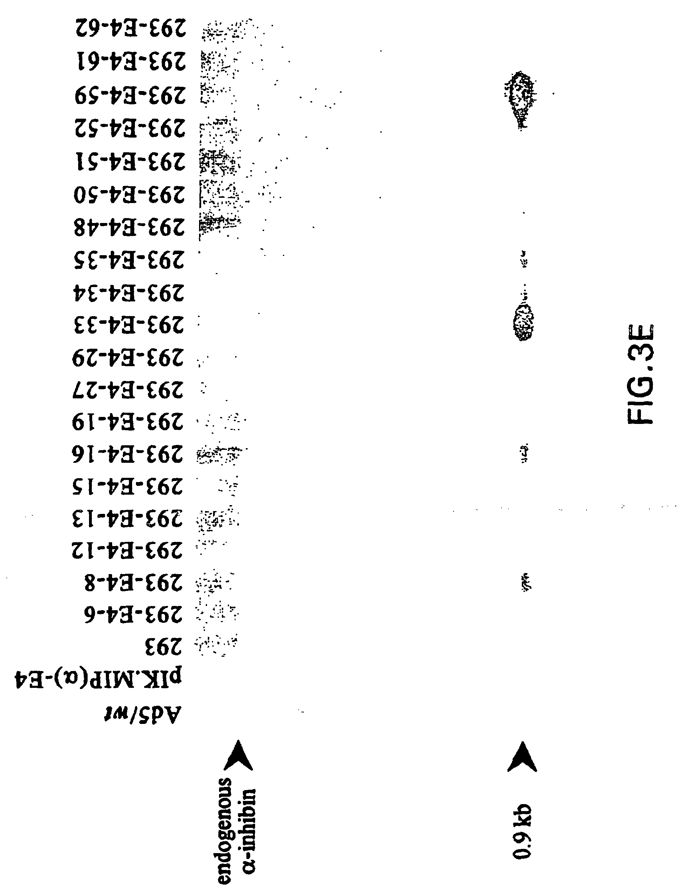

To examine the integration of the introduced adenovirus E4 region, genomic DNA from each clone was digested with either Hind III and Sfi I, or Nco I restriction enzymes and analyzed by Southern transfer. Fig. 3A shows a restriction map of the introduced α-inhibin-E4 region and corresponding regions of the E4 probe (Sma I H fragment of Ad5) and the inhibin promoter probe. 17 clones out of a total of 66 presented the correct DNA patterns as predicted for a full length E4 region DNA integration in the screen blots of both digestions. Other clones showed either no integration or integration with variable sizes of E4 region. Fig. 3B-3E represent the Southern blots of genomic DNA extracted from the 17 clones with full length integration and two clones which contains variable sizes of E4 region integration on the initial screening blots. The DNA was extracted after maintaining these 19 cell lines in the non-selective medium for more than 30 passages. As shown in Fig. 3B and 3C, 15 cell lines represent the characteristic 0.9 kb and 3.2 kb fragments in HindIII/Sfi I digestion and 1.6 kb and 2.1 kb fragments in Nco I digestion. There were no detectable E4 region sequences in two cell lines (lines 13 and 29) which had the same integration patterns as the other 15 lines in the screening blots, indicating an unstable integration event in these two lines. Lines 16 and 19 are examples of cell lines which retained the E4 gene region with variable restriction patterns. The 0.9 kb band of all 15 lines hybridized to the mouse inhibin promoter sequence in the Hind III/Sfi digestion (Fig. 3D). The 3.1 kb fragment along with the 2.1 kb fragment was hybridized to the inhibin promoter probe in the Nco I digestion blot. These results indicate that a full length gene region of E4 was stably integrated into these 15 cell lines. To rule out the possibility that these cell lines can survive and maintain a full length of the E4 region due to a loss of the E1 gene region, the blots were reprobed with the Ad5 Hind III E fragment (m.u. 7.7-17.1). All 19 lines have a same sized fragment detected by the E1 probe as that in the parental 293 cell line (Fig. 3e). Therefore, the E1 gene was not altered in the 293-E4 cell lines.

### Example 10

### Screen of biological activity of 293-E4 cell lines

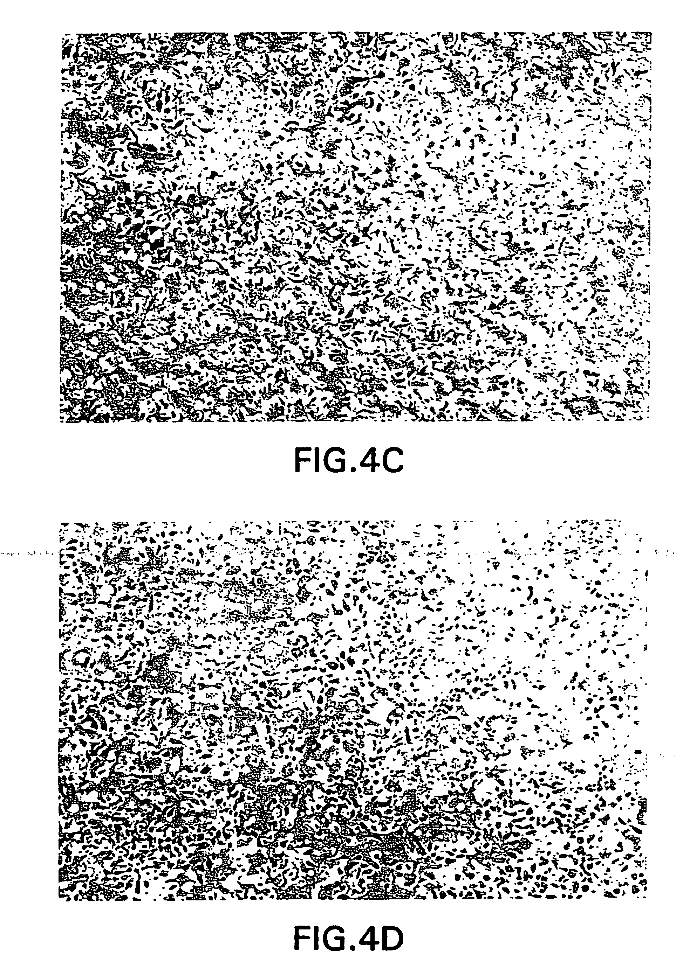

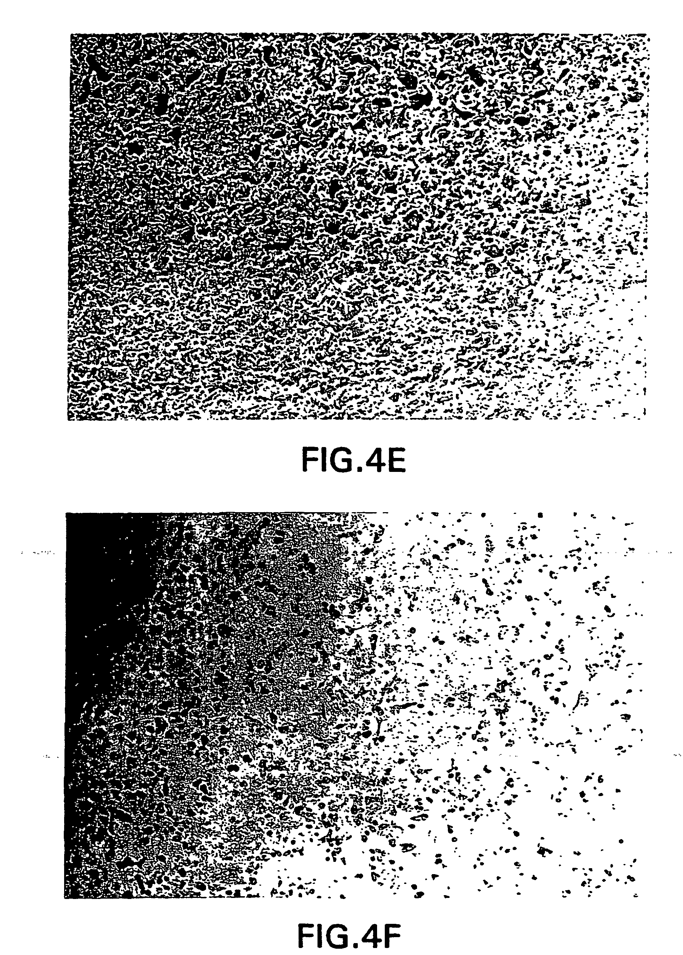

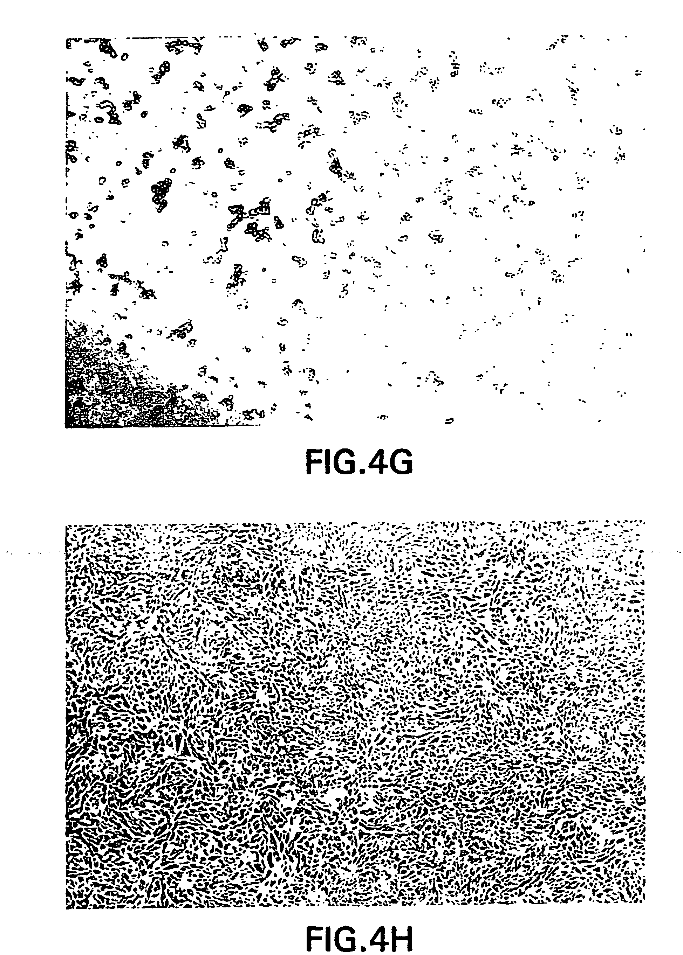

To determine whether these cell lines were capable of supporting the E4 deletion virus growth, each of the cell lines was infected with an adenovirus E4 deletion mutant virus H5dl1014 [Bridge & Ketner, (1989)]. The E4 defective strain H5dl1014 contains two deletions from m.u. 92 to 93.8 and m.u. 96.4 to 98.4. The deletions destroy all the open reading frames of the E4, region except ORF 4. This virus produces substantially less viral DNA and late viral proteins in Hela cells similar to that seen in cells infected with H2dl808 and H5dl366 [Halbert, et al, *J. Virol*. **56:** 250-256 (1985)]. The only permissive cell line for the growth of H5dl1014 is W162 [Weinberg & Ketner, (1983)]. When the parental 293 cells, W162 cells and all 15 lines were infected with H5d11014 at m.o.i. 25 with or without addition of the 1mM cAMP, 6 cell lines showed comparable cytopathic effect (CPE) as observed on W162 cells at 3-4 days of post-infection (Fig. 4). The CPE appeared much faster in the presence of cAMP both in W162 cells and in some of the 293-E4 cell lines. The parental 293 cells showed CPE at much milder level (Fig. 4). This result shows that 293-E4 cell lines (containing both E1 and E4 gene regions) support the growth of E4 deleted viruses (eg., H5dl1014 virus) as efficiently as cell lines containing the E4 gene region only (eg., W162 cell line).

### Example 11

### Induction of H5dl1014 production on 293-E4 cell lines

To quantitatively examine the ability of 293-E4 cell lines to produce H5dl1014 mutant virus and to determine whether there is a specific induction of E4 gene expression in the 293-E4 cell lines, the titer of the H5dl1014 produced from the 293-E4 cell lines was measured in the presence or absence of cAMP. Viral stocks were prepared from each cell line by infecting the same number of cells with H51014 at m.o.i. 50. At 48 hr post-infection, the supernatant of each cell line was removed and the cells were resuspended in 1/10 of the original volume of serum free medium. Titration of the viral stocks were performed on W162 cells by plaque assay. As presented in Table 1, the phenomenon of virus production from these 15 lines can be generally classified into three groups. Group 1 which includes lines 8, 50 and 51 showed increased viral titers by 4 to 6 orders of magnitude compared to the titer produced from 293 cells. Line 8 and 51 had a 10 fold increase of the viral titers in the presence of CAMP. Group 2, which includes lines 12, 27 and 61, produced similar titers of virus as that produced from W162 cells. The titers increased 1,000-10,000 fold with the exception of line 12 in which the level of virus production increased by 7 orders of magnitude in the presence of cAMP. These results indicate an induced E4 gene expression in these three cell lines. Group 3 includes the remaining cell lines which produced the virus titers essentially at levels similar to that produced from parental 293 cells in the presence or absence of cAMP. The induced E4 gene expression is also indicated in several cell lines in this group.

The 10 fold induction was also observed in the W162 cells and parental 293 cells when the cells were treated with cAMP. It is possible that this 10 fold increase in the virus yield is due to the enhancement effect of cAMP on other adenovirus early gene expression [Leza & Hearing, *J. Virol.* **63:** 3057-3064 (1989)] which also contains CRE elements causing an increase in viral DNA synthesis.

**TABLE IV Titers of H5d11014 produced from cell lines W162, 293, and 293-E4**

| GROUP | CELL LINE | TITER [pfu/ml]^{†} | |
|---|---|---|---|
| | | No cAMP | 1mM cAMP |
| control | W162 | 22x10¹³ | 1.2x10¹⁴ |
| | 293 | 1.6x10⁴ | 2.7x10⁵ |
| 1 | 293-E4-8 | 8.9x10¹² | 33x10¹³ |
| | 293-E4-50 | 6.7x10¹⁰ | 4.5x10¹⁰ |
| | 293-E4-51 | 8.9x10² | 22x10⁹ |
| 2 | 293-E4-12 | 45x10⁵ | 8.9x10¹² |
| | 293-E4-27 | 6.7x10⁹ | 2.2x10¹³ |
| | 293-E4-61 | 1.3x10₁₀ | 8.0x10¹³ |
| 3 | 293-E4-6 | 1.1x10⁴ | 8.9x10⁴ |
| | 293-E4-15 | 1.3x10₅ | 6.7x10⁶ |
| | 293-E4-33 | 6.7x10₄ | 1.6x10⁶ |
| | 293-E4-34 | 6.7x10⁶ | 1.3x10⁷ |
| | 293-E4-35 | 1.3x10⁵ | 1.1x10⁶ |
| | 293-E4-48 | 6.7x10⁴ | 6.7x10⁶ |
| | 293-E4-52 | 1.8x10⁴ | 1.3x10⁷ |
| | 293-E4-59 | 3.3x10³ | 6.7x10⁶ |
| | 293-E4-62 | 1.6x10⁵ | 6.7x10⁶ |

| | | | |
|---|---|---|---|
| ^{†}The titer was determined by plaque assay on W162 monolayer culture. | | | |
| Values in the table are the averages of titers measured on duplicate samples. | | | |

### Example 12

### Generation of Ad5/ΔE1 (β-gal)ΔE4 virus

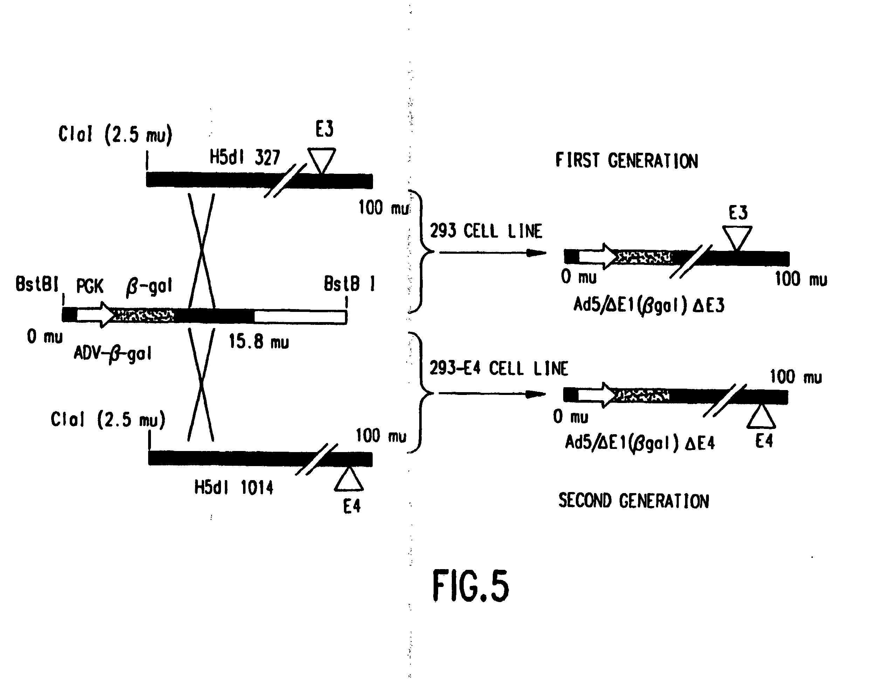

To rescue recombinant virus which harbors lethal deletions in both the E1 region and the E4 region the two most efficient cell lines, line 8 and line 61, were utilized. The ADV-β-gal plasmid was linearized by BstBl and co-transfected with Cla I digested H5dl1014 into the monolayers of 293-E4 cell lines (Fig. 5). The recombinant virus was generated by *in vivo* recombination between the overlapping adenoviral sequence of ADV-β-gal and the H5dl1014 large Cla I fragment (m.u. 2.55-100). Plaques appearing at 7-10 days post-transfection were isolated and purified by blue plaque assay. The final purified blue plaque and the viral DNA were analyzed (Fig. 6). For the following comparative studies of the double deletion recombinant virus, the Ad5/ΔE1(β-gal)ΔE3 virus was generated. This virus was generated by co-transfection of Bst BI linearized ADV-β-gal plasmid with Cla I digested H5dl327 [Thimmappaya, et al, (1982)] into 293 cells (Fig. 5).

### Example 13

### In vitro evaluation of the Ad5/ΔE1(β-gal)ΔE4 virus

To evaluate the infectivity of this second generation of recombinant virus, infectivity was compared with the β-gal gene expression of the double lethal deletion virus and single lethal deletion virus in Hela, 293, W162 and line 61 cells. The cells were infected with these two strains of recombinant viruses at 20 m.o.i. for 48 hrs. Expression was observed in both infections as detected both by histochemical staining and the β-galactosidase activity assay described *supra*. The abolished cytopathic effect of the Ad5/ΔE1(β-gal)ΔE4 virus was also tested by the plaque assay. The 293-E4 was the only permissive cell line for all three strains of virus (Ad5/ΔE1(β-gal)ΔE4, Ad5/ΔE1(β-gal)ΔE3 and H5dl1014). The 293 cells were permissive for the Ad5/ΔE1(β-gal)ΔE3 virus, semi-permissive (low level of virus production) for the H5dl1014 virus but non-permissive to Ad5/ΔE1(β-gal)ΔE4 virus. The W162 cell line was permissive for H5dl1014 virus, but non-permissive for Ad5/ΔE1(β-gal)ΔE3 virus and Ad5/ΔE1(β-gal)ΔE4 virus. Hela cells are non-permissive for all three strains of viruses. These results demonstrate that the double deletion virus does not cause any cytopathic effect to the human cell lines tested. Absence of cytopathic effects following infection of the double deletion viruses at m.o.i. 20 suggests that in vivo these viruses will not express late gene products. This should eliminate the immune response against cells infected with recombinant virus, thereby prolonging transgene expression.

### Example 14

### Efficient transgene expression and truncated E4 gene expression in vitro

To determine the transgene expression mediated by Ad5/ΔE1(β-gal)ΔE4 at the transcription level and physically visualize the E4 transcription from the Ad5/ΔE1(β-gal)ΔE4 virus, Ad5/ΔE1(β-gal)ΔE4 viral RNA was analyzed by Northern blot. Total RNA was harvested from Hela cells at 4, 24 and 48 hr following infection of recombinant adenoviruses at 20 pfu/cell. Total RNAs extracted from Hela cells infected with H5d1327 and Ad5/ΔE1(β-gal) were used as comparison. RNAzol B reagent (Tel-Test, Inc. Friendswood, TX) was used for extraction of total RNA. Ten ug total RNAs were electrophoresed in a 1% denaturing gel, transferred to a membrane filter, and hybridized to radioactive DNA probes. The Northern blots were sequentially probed with radiolabled 1.65 kb EcoRV-AccI fragment of β-gal, 2.30 kb SmaI H fragment of Ad5 E4 region (m.u. 92..0-98.4), 765 bps of the PCR product of the L5 region and the 1.45 kb SmaI I fragment of the L3 region (m.u. 52.6-56.6). The PCR primers for amplification of adenovirus L5 region were 5'-GAGGACTAAGGATTGATT-3' (NTs 31811-31828) (SEQ ID NO: 3) and 5'-CGTGAGATTTTGGATAAG-3' (NTs 32549-32566) (SEQ ID NO:4).

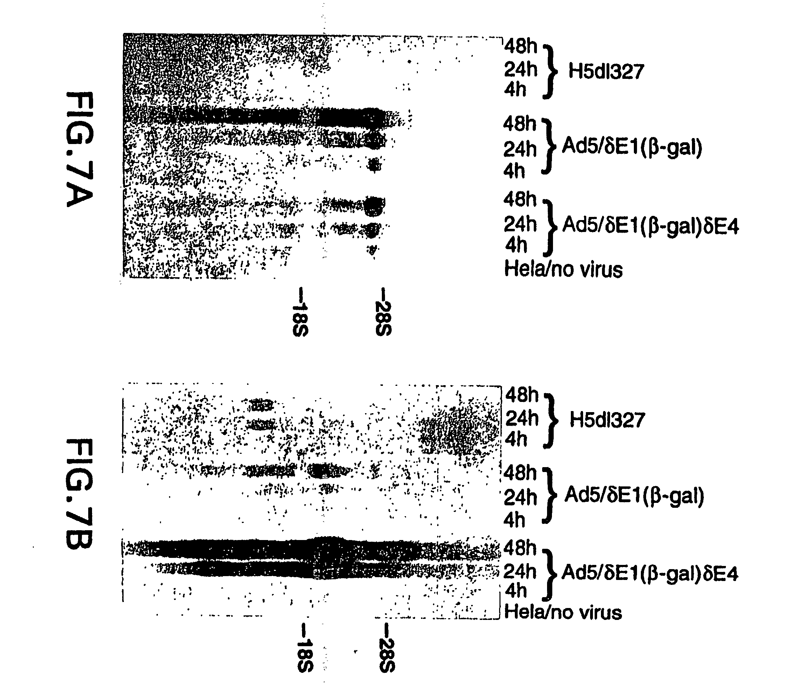

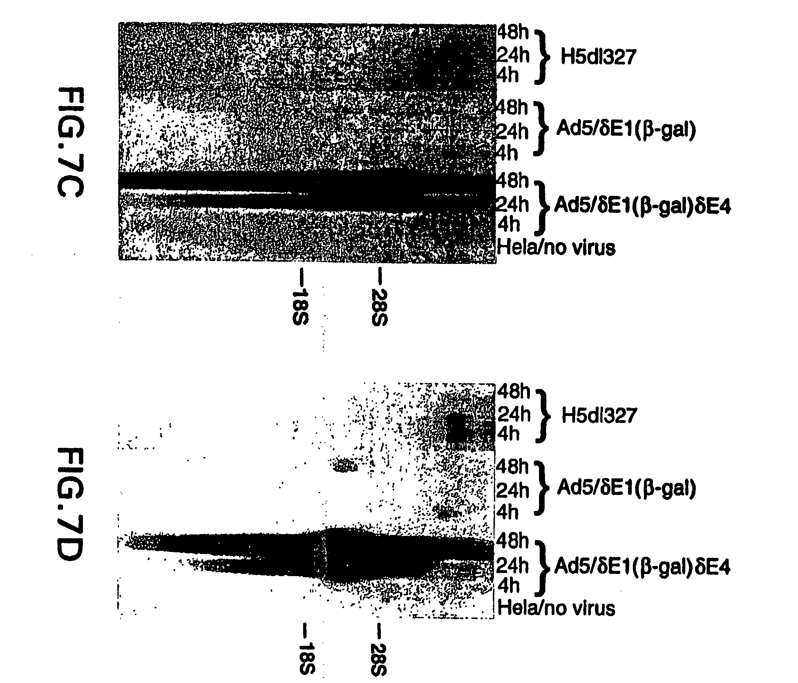

The cells infected by either the Ad5/ΔE1(β-gal)ΔE4 or the Ad5/AE1(β-gal) accumulated same level of β-gal mRNA at 4 hr post-infection (Figure 7, Panal A). However, the cells infected with Ad5/ΔE1(β-gal)ΔE4 gradually accumulated lower level of β-gal at 24 and 48 hr post-infection compared to the cells infected with Ad5/ΔE1(β-gal). This slightly reduced level of β-gal transcript mediated by Ad5/ΔE1(β-gal)ΔE4 is consistant with a slightly reduced level of β-galactosidase enzyme activity in infected Hela cells assayed at 24 hr post-infection as previously described. When the same blot was rehybridized with adenoviral E4 probe which extends from 92.0 to 98.4 m.u. and does not overlap the 3' end of the L5 region [Fraser, et al, *J. Mol. Biol.* **155:** 207-233, (1982)], a characteristic pattern of polysomal mRNAs [Tiggs, et al, *J. Virol.* **50**: 106-117, (1984)] was displayed in both H5d1327 and Ad5/ΔE1(β-gal) infected samples although the level of the E4 transcripts is dramatically decreased in Ad5/ΔE1(β-gal) infected cells. However, there is only one species of E4 transcripts at a size corresponding to 1.5 kb in cells infected with the Ad5/ΔE1(β-gal)ΔE4 (Figure 7, Panal B). This observation is presumably due to two large deletions within this E1/E4 deleted vector which destroyed all the open reading frames within the E4 region with the exception of the ORF4 and resulted in the production of a truncated transcripts encoding the ORF4 protein. This example supports the results described in Example 13 that the transgene delivered by the Ad5/ΔE1(β-gal)ΔE4 recombinant adenoviral vector is efficiently expressed.

### Example 15

### Reduced or eliminated adenviral late gene expression in vitro

The parental mutant adenovirus H5dl1014 which was used to generate the recombinant adenoviral vector Ad5/ΔE1(β-gal)ΔE4 has been reported to show severe defects in late gene expression [Bridge and Ketner, *J*. *Virol*. **63:** 631-638, (1989)]. To determine whether the combination of the E1 and E4 region deletions in Ad5/ΔE1(β-gal)ΔE4 might result in more profound defects or complete blockage of late gene expression, the accumulation of late mRNAs of Ad5/ΔE1(β-gal)ΔE4 in nonpermissive Hela cells was measured by both Northern blot and reverse transcription polymerase chain reaction (RT-PCR) methods. The Northern blot (described in Example 14 and Figure 7) was rehybridized to the L5 probe, which is the PCR product of Ad5 sequence from NTs 31811 to 32566 within the fiber protein coding region (L5 region), and the L3 probe, which is the SmaI I fragment from m.u. 52.6-56.6 within the hexon protein coding region. There was a low level of accumulation of L5 transcripts and a detectable level of L3 mRNA in the cells infected with E1-deleted vector at 48 hr post-infection (Figure 7, Panal C and D). However, both late transcripts were not detectable in the cells infected with the E1/E4-deleted adenoviral vector.

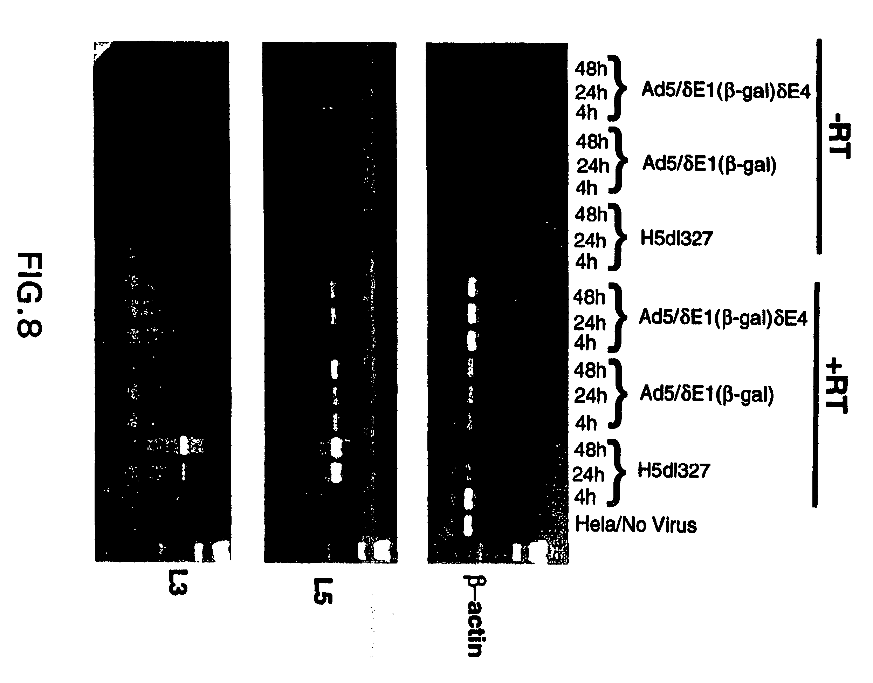

Applicants further employed the RT-PCR method with increased detection sensitivity to determine whether adenoviral late gene transcripts were expressed in the Ad5/ΔE1(β-gal)ΔE4 recombinant vector. Total RNA was treated with RNase-free DNase (promega Corp., Madison WI) at 1 unit/ug at 37 °C for 60 min. The first strand of cDNA was synthesized using pd(N)₆ as primer (Pharmacia, Alameda, CA). The same set of control reactions was done by omitting the reverse transcriptase. Both preparations (RT+ and RT-) were then amplified using the same L5 primers as described previously (see Example 14). The primers for L3 region were 5'-CCTACGCACGAC-3' (SEQ ID NO: 5)(NTs 18996-19007); 5'-TGTTTGGGTTAT-3' (SEQ ID NO: 6) (NTs 20318-20329). After amplification, the RT products wee run on a 1% agarose gel and visualized by ethidium bromide staining. The L5 mRNA was identified both in cells infected with Ad5/ΔE1(β-gal)and Ad5/ΔE1(β-gal)ΔE4 (Figure 8). There was no detectable L3 mRNA transcripts in cells infected with Ad5/ΔE1(β-gal)ΔE4 (Figure 8). The RT-PCR reaction using β-actin primers was used as an internal control. The β-actin primers utilized for the RT-PCR were the consensus sequences between the rat and human as described in Fraser, et al, *J. Virol.* **63,** 631-638, (1989).

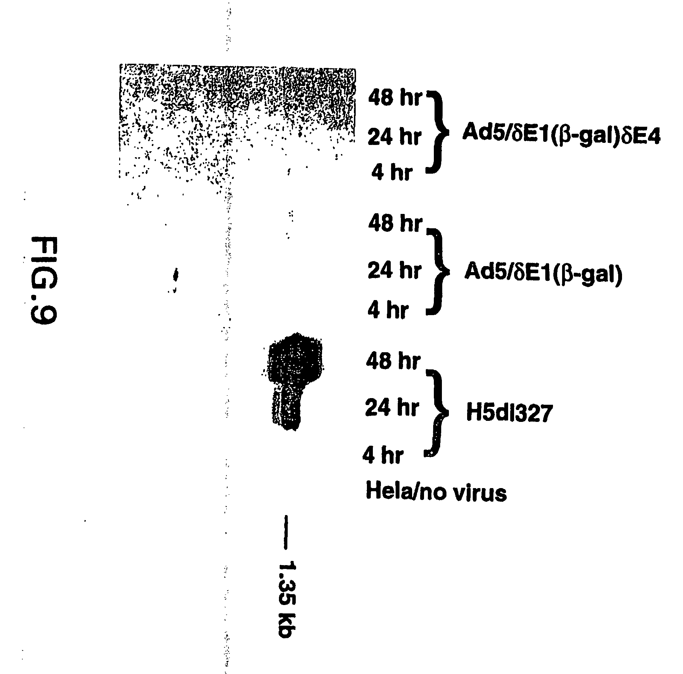

To increase the sensitivity of detection of the hexon protein sequence (within the L3 region), RT-PCR products were further analyzed by Southern blot probed with an oligomer 5'-GACCG7GAGGATACT-3 - (SEQ ID NO: 7) which hybridized to the internal region of the RT-PCR products of the hexon protein coding region (Figure 9). L3 transcripts were not detected in the cells infected with the double deleted Ad5/ΔE1(β-gal)ΔE4 adenoviral vector which confirms the results of the study described above and in Figure 8. These results indicate that the combination of the E1 and E4 deletions within the Ad5/ΔE1(β-gal)ΔE4 vector should result in a complete deficiency of the L3 mRNA which encodes the adenovirus capsid protein-hexon.

### Example 16

### Persistent transgene expression in vivo

To determine whether reduced or eliminated adenovirus late gene expression of the E1/E4 deleted adenoviral vector could prolong transgene expression, the β-gal gene expression in cells infected with either E1-deleted vector or E1/E4-deleted vector was examined. The double deleted Ad5/ΔE1(β-gal)ΔE4 recombinant virus and Ad5/ΔE1(β-gal) recombinant virus were used in the following *in vivo* experiments. Viral stocks were produced from suspension of complementing packaging cells and purified by double CsCl banding as described in Graham and Prevec, *Methods Mol. Biol.* **7**: 109-128, (1991). All the stocks used were free of contamination of E1-containing virus determined by PCR analysis using E1 region (NTs 13-1338) primer and E2 region (NTs 5053-5072)primer as described in Lochmuller, et al, *Hum. Gene Ther.* **5**: 1485-1491, (1994). Five animals infected with each strain of recombinant virus were sacrificied at day 3, 7, 14, 21, 28, 35 and 77 postinfection. X-gal histochemical staining, described previously, was performed on the frozen sections of the above infected animal livers. The staining showed that approximately 100% of tissues expressed the β-galactosidase gene in both E1-deleted and E1/E4-deleted adenoviral vector infected livers at day 3 and 7. There was a sharp declining of X-gal staining from 14 days (75-85%) to 35 days (15-25%) in the livers infected with the El-deleted vector. At 77 days, only 1-2% of the livers stained blue in El-deleted adenovirus infected animals. In contrast, β-galactosidase gene expression was sustained at a level of 85% for 28 days in the livers infected with the E1/E4-deleted virus. Moreover, at day 77, approximately 65-75% of the E1/E4-deleted adenovirus infected animal livers expressed the β-galactosidase gene. This example demonstrates that the elimination of the adenovirus late gene expression in a E1/E4 double deleted adenoviral vector (adenovirus) could significantly prolong the expression of a transgene placed in the viral vector compared to a single deleted adenovirus, e.g., E1 deleted adenovirus.

### Example 17

### Reduced cytopathic effects and host immune responses in vivo

To determine whether there is an inverse correlation between a prolonged transgene expression and reduced cytopathic effects in animals infected with the E1/E4-deleted adenovirus, random liver hematoxylin/eosin (H&E) stained sections from five animals per each experimental group were examined. Frozen liver section (6 um) were fixed in 0.5% glutaraldehyde and stained for β-gal activity by staining in X-gal solution. For morphological study, the paraffin liver sections were stained with H&E. Random sections were reviewed. Pathological changes such as cell ballooning, tissue necrosis, loss of lobular structure and inflammatory infiltration were observed between day 3 and 7 and continued through day 35 in animals infected with E1-deleted adenovirus vector. By day 77, most animals with same infection were recovered from these tissue damages morphologically. However, none of the above pathologic changes was observed between day 3 and 7 except a slight inflammatory infiltration appeared after day 14 and in the animals infected with E1/E4-deleted adenovirus vector. By day 77, all the animals infected with this doubly deleted virus vector were retured to normal morphologically. This example demonstrates that reduced cytopathic effects are mediated by the double deleted Ad5/ΔE1(β-gal)ΔE4 recombinant adenoviral vector. The prolonged transgene expression in animals infected with the double deleted adenoviral vector may be due to decreased tissue regeneration activity in the liver compared to the livers of animals infected with the Ad5/ΔE1(β-gal) vector.

### Example 18

### Construction E4-ORF-6 plasmid

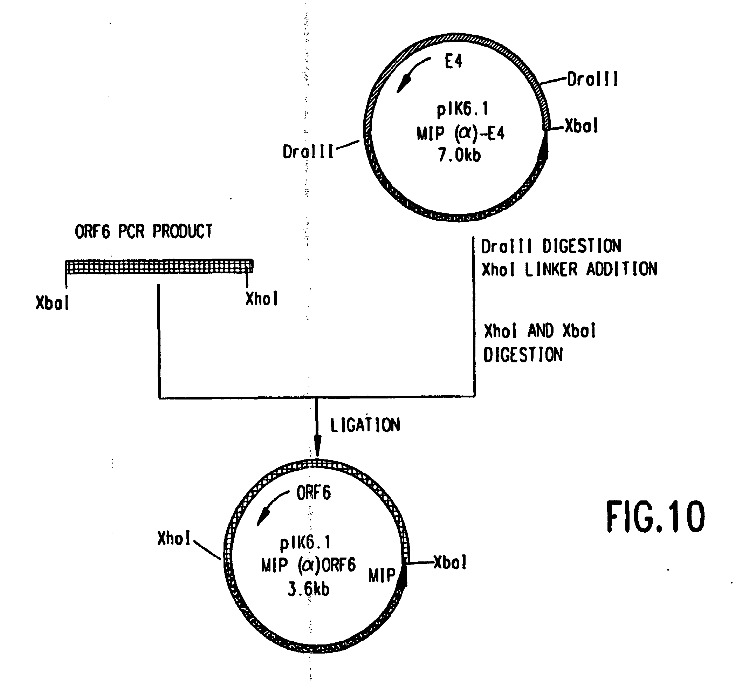

This example describes the construction of pIK6.1MIP(α)-ORF6 plasmid. The E4-ORF6 region expression vector was constructed as illustrated in Figure 10. The parental pIK6.1 MMSV-E4 (ΔE4 pro.) derived from the pIK6.1.MMSVNhe [also referred to as pIK6.1 MMSVenpoNhe(Hpa) or pkat1 in Finer, et al, Blood, 1994 and Finer, et al, in International application WO 94/29438] contains the full length sequence of the E4 region except the promoter sequence. The pIK6.1MIP(α)-E4 was constructed by ligation of a 238 bp fragment of the Hind III-Xba I PCR product of mouse α inhibin promoter [MIP(α)] with the 2.9 kb Xba I-Stu I fragment and the 3.9 kb Stu 1-Hind III fragment of the pIK6.1 MMSV-E4 (ΔE4 pro.). The pIK6.1MIP(α)-ORF6 plasmid was constructed by replacing the promoterless E4 region with a PCR product of the ORF6 fragment. The PCR primers for the E4-ORF6 coding region are 5'-gccaatctagaGCTTCAGGAAATATGACT-3' (Ad5 NTs 34072 to 34089)(SEQ ID NO:8) and 5'-catctctcgagGGAGAAGTCCACGCCTAC-3' (Ad5 NTs 33179 to 33196) (SEQ ID NO:9). The sequences containing either the XhoI site or Xba I site in lowercase were present to facilitate cloning. The transcription of the ORF6 is driven by the mouse α inhibin promoter and the heterologous polyadenylation signals (SV40 polA) on the plasmid backbone downstream of the ORF6 region was utilized. The cloned ORF6 DNA fragment was sequenced to verify the accuracy of the sequence. The pIK6.1MIP(α)-ORF6 was used to generate the packaging cell line as described infra.

### Example 19

### Construction of 293-ORF6 cell lines

The following example describes the construction of 293-ORF6 cell lines To eliminate the potential possibility of generating E4 containing virus, this new packaging cell line has been established by introducing a minimum essential Ad5 E4 ORF6 coding region into 293 cells. The plasmid pIK. MIP(α)-ORF6 carries a 910 bp PCR fragment of Ad5 E4-ORF6 coding region from nucleotide 1846 to 2756 numbered from the right end of the genome. The ORF6 region was cloned downstream of the mouse α inhibin promoter region as previously described. The pIK6.IMIP(α)-ORF6 was co-transfected into 293 cells with a plasmid containing the Neor gene. Fifty-four G418 resistant clones were isolated, expanded and screened for integration of the E4-ORF6 sequence by Southern blotting (Figure 11). Genomic DNA from each clone was digested with Hind III and XmnI and hybridized to the ORF6 PCR fragment (Figure 11, Panel A). Eight out of the total 54 screened clones retained at least one copy of predicted 1.7 kb fragment for intact ORF6 region. The blots were rehybridized with the E1 probe which is a Ad5 Hind III E fragment (m.u. 7.7-17.1) (Figure 11, Panel B). All eight 293-ORF6 cell lines showed the same sized fragment detected by the E1 probe as that found in the parental 293 cells (Figure 11). This example demonstrates that the structure of the E1 gene has not been altered in the cell lines. Not only do the cell lines above have the intact E1 gene but they also retain at least one copy of the E4 ORF6 region.

### Example 20

### Complementation of E4 function by 293-ORF6 cell lines

The 293-ORF6 cell lines were screened for their ability to produce virus following infection with the E4-deleted mutant adenovirus, H5dl1014. The H5dl1014 adenovirus contains two deletions which destroy all the open reading frames of the E4 region with the exception of ORF4, resulting in the production of substantially less viral DNA and late viral proteins in Hela cells. The W162 cell line, which contain intact E4 region, is a permissive cell line for the growth of H5dl1014 [Bridge and Ketner, *J. Virol*. **63**: 631-638, (1989)]. When the parental 293, W162, 293-E4 and 293-ORF6 cell lines were infected with H5dl1014 at an moi of 25 pfu, all eight 293-ORF6 cell lines showed comparable cytopathic effect (CPE) as observed in W162 cells as well as in 293-E4 cells at 3-4 days of post-infection. Quantitative analysis of the production of H5dl1014 was performed by plaque assay with limiting dilution on the monolayers of the 293-ORF6 and control cell lines. The titers of the H5d11014 produced by 293-ORF6 are at similar range of that produced from both W162 and 293-E4 cells. (Table V) Thus, 293-ORF6 cell lines which only contain a small essential DNA fragment of the E4 gene region are sufficient to complement the E4 function and support the growth of the E4 deletion mutant virus.

### Example 21

### Complementation of E1 function by 293-ORF6 cell lines

Southern analysis demonstrated that all of the 293-ORF6 cell lines examined contain an intact E1 region copy. These lines were assayed for their biological activity to complement the E1 function. (Complementary activity assay as shown in Table V.) Monolayers of W162, 293, 293-E4, and 293-ORF6 #34 cell lines were infected with the E1-deleted mutant virus, H5dl312 and viral production was determined by limiting dilution plaque assay. Each of the eight 293-ORF6 cell lines produced the E1-deleted virus at a level similar to that produced by the parental 293 cells (Table V). Therefore, the 293-ORF6 cell lines possess the ability to complement both the E1 and E4 gene product functions.

**TABLE V Characterization of E4-ORF6 cell lines by biological complementation activity**

| Cell Line | Titer (pfu/ml)^{c} | | |
|---|---|---|---|
| | dl1014^{a} | dl312^{b} | ΔE1/ΔE4^{b} |
| W162 | 5.0 x 10⁷ | 0 | 0 |
| 293 | 0 | 2.2 x 10¹⁰ | 0 |
| 293-E4 | 6.0 x 10⁶ | 1.8 x 10¹⁰ | 2.0 x 10⁶ |
| ORF6-34 | 6.0 x 10⁷ | 6.0 x 10¹⁰ | 5.0 x 10⁶ |

| | | | |
|---|---|---|---|
| ^{a}The titers of H5d11014 lysates produced from cell lines were determined by plaque assay on W162 monolayer culture. | | | |
| ^{b}The titers of H5d312 stock and dE1/dE4 stock were determined on cell lines. | | | |
| ^{c}The values in the table are the averages of titers measured on duplicate samples. | | | |

### Example 22

### Simultaneous complementation of both E1 and E4 functions by 293-ORF6 cell lines

This example describes the ability of the 293-ORF6 cell lines to rescue recombinant virus which harbors deletions in both the E1 and the E4 regions. Cell line 34 was chosen for further testing from the two cell lines which produced the highest titer of H5dl1014, i.e., cell line 21 and 34. The E1/E4 double deleted recombinant virus, Ad5/ΔE1(β-gal)ΔE4, constructed as described previously, contains the *E*. *coli* β-galactosidase gene which is under the control of *pgk* promoter. Ad5/ΔE1(β-gal)ΔE4 was generated by recombination using H5dl1014 as parental virus as previously described. Quantitative analysis of the production of Ads/ΔE1(β-gal)ΔE4 was performed by plaque assay with limiting dilution on the monolayers of the control cell lines and 293-ORF6-34 line. Plaques which stained blue with X-gal staining appeared on the monolayers of 293-E4 and 293-ORF6-34 at 7-10 days post-infection. The titer of the Ad5/ΔE1(β-gal)ΔE4 produced from 293-ORF6-34 cell line was the same as the titer produced from the 293-E4 cell line. This example demonstrates that the 293-ORF6-34 cell line is able to support the growth of the virus having both E1 and E4 lethal deletions. The new packaging cell lines described in Example 19 are advantageous for the propagation of E1/E4-deleted recombinant adenoviral vectors because they produce high titer virus and are unable to generate replication-competant adenovirus (RCA) due to the absence of overlap between the E4 deletion within the vector and the E4-ORF6 expressing plasmid present in the transfected cell line.

### Example 23

### Construction of E2A plasmid

The pIK6.1MIP(α)-E2A plasmid was derived from the pIK6.1MIP(α)-E4 as described above. The promoterless E4 gene was replaced with the Ad5 E2A gene from 21562 to 24627 (m.u. 59.9 to 68.3) [Klessig, et al, *Mol. Cell. Bio.* **4**: 1354-1362, (1984)] with the second leading sequence present. The Ad5 E2A gene encodes the adenovirus DNA binding protein (DBP) and is required for adenovirus DNA replication [Van er Vliet and Sussenbach, *Virology,* **67**: 415-426, (1975)]. The gene (m.u. 61.5-68) which lacks its own promoter and the first leader sequence was cloned downstream of the mouse α inhibin promoter region. A PCR product from m.u. 65.2 to 68.3 was generated using primers 5'-tccatttctagaTCGGCTGCGGTTG-3' (SEQ ID NO: 10) (Ad5 NTs 24615 to 24627) and 5'-ACGTGGTACTTGTCCATC-3' (SEQ ID NO: 11) (Ad5 NTs 23443 to 23460). The sequence containing the Xba I site in lowercase is present to facilitate ligation and cloning. The PCR product was digested with both Xba I and Pvu I, and ligated with the Ad5 Bam HI and Pvu I DNA fragment from NTs 21562 to 23501 (m.u. 59.9 to 65.2). The promoterless E2A DNA sequence was next used to replace the promoterless E4 region on the plasmid pIK6.1MIP(α)-E4. The transcription of the cloned E2A gene is driven by the mouse α-inhibin promoter and the heterologous polyadenylation signals (SV40 polA) on the plasmid backbone downstream of the E2A region was utilized. The cloned E2A gene was sequenced to verify the accuracy of the sequence. The pIK6.1MIP(α)-E2A plasmid was used to generate the packaging cell line as described *infra*.

### Example 24

### Construction of 293-E2A cell line

The following example describes the construction of the 293-E2A cell lines. To construct a packaging cell line which is able to complement both the E1 and the E2A gene functions in trans simultaneously, the plasmid pIK. MIP(α)-E2A was cotransfected into 293 cells with a plasmid containing the Neo^{r} gene. The 293 cells (ATCC CRL1573) were grown in Dulbecco's modified Eagle's medium (DMEM), 1g/L glucose (JRH Biosciences, Denver, PA), 10% donor calf serum (Tissue Culture Biologics, Tulare, CA). Cells were seeded at 5x10⁵ per 10-cm plate 48 hours prior to the transfection. Ten mg of pIK6.1MIP(a)-ORF6 and 1 mg of pGEM-pgkNeo.pgkpolyA, encoding the neomycin resistance gene under the control of the mouse phosphoglycerate kinase promoter were co-transfected into 293 cells by calcium phosphate co-precipitation [Wigler, et al, *Cell* **16**: 777-785, (1979)].

Fifty G418 resistant clones were isolated, expanded and screened for integration of the E2A sequence by Southern blotting. Genomic DNA from each clone was digested with Xba I and Afl II and hybridized to the E2A probe. Twelve out of the total 50 screened clones retained at least one copy of predicted 1.44 kb fragment for intact E2A region. The blots were reprobed with the E1 probe (Ad5 Hind III E fragment from m.u. 7.7-17.1). All twelve 293-E2A cell lines have a fragment with same size as that in the parental 293 cells. This example demonstrates that the structure of the E1 gene has not been altered in these cell lines and that the cell lines retain at least one copy of the E2A gene.

### Example 25

### Construction of 293-E4/E2A cell line

The following example describes the construction of the 293-E4/E2A cell lines. To construct a packaging cell line which is able to complement the functions of the E1, the E2A and the E4 in trans simultaneously, the plasmid pIK. MIP(α)-E2A was cotransfected into 293-E4 cells with a plasmid containing the Neo^{r} gene. The 293-E4 cells were grown in Dulbecco's modified Eagle's medium (DMEM), 1g/L glucose (JRH Biosciences, Denver, PA), 10% donor calf serum (Tissue Culture Biologics, Tulare, CA). Cells were seeded at 5x10⁵ per 10-cm plate 48 hours prior to the transfection. Ten mg of pIK6.IMIP(α)-ORF6 and 1 mg of pGEM-pgkNeo.pgkpolyA, encoding the neomycin resistance gene under the control of the mouse phosphoglycerate kinase promoter were co-transfected into 293 cells by calcium phosphate co-precipitation [Wigler, et al, 1979]. Fifty G418 resistant clones were isolated, expanded and screened for integration of the E2A sequence by Southern blotting. Genomic DNA from each clone was digested with Xba I and Afl II and hybridized to the E2A probe. Twenty-one out of the total 50 screened clones retained at least one copy of predicted 1.44 kb fragment for intact E2A region. The blots were reprobed with the E1 probe (Ad5 Hind III E fragment from m.u. 7.7-17.1) and E4 probe (Smal H fragment from m.u.92-98.4). All twenty-one 293-E2A cell lines have the same integrated E1 and E4 DNA patterns as those of their parental cell line-293-E4. This example demonstrates that the structures of the E1 and E4 genes have not been altered in these cell lines and in addition, one copy of the E2A gene is retained in all of these lines.

### Example 26

### Construction of virus-associated RNA (VARNA) plasmid

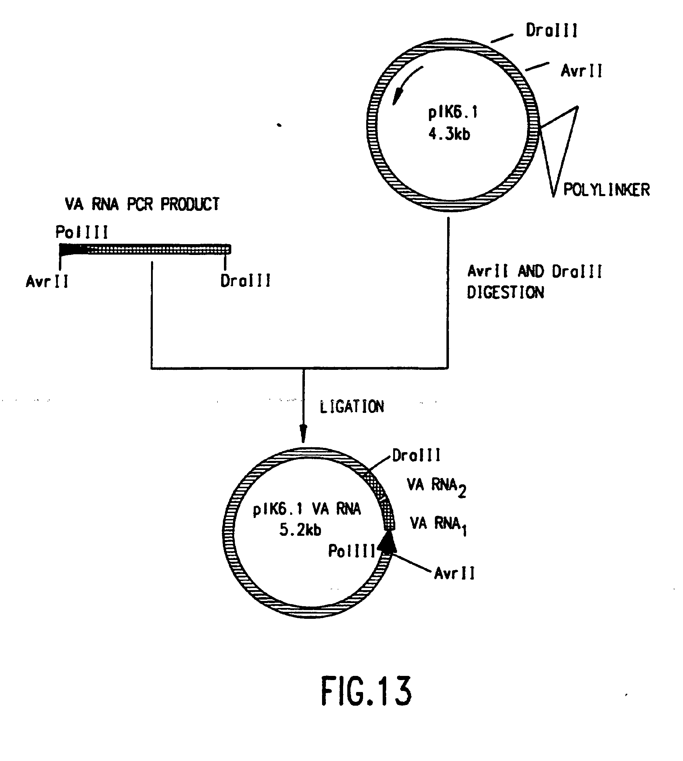

This example describes the construction of the pIK6.1-VARKA plasmid. The pIK6.1-VARNA plasmid was derived from the pIK6.1 which was previously described by Finer et al in WO 94/29438. A PCR product which contains Ad5 VA RNA1 and VA RNA2 genes with their endogenous promoter for RNA polymerase III from m.u. 29 to 30.1 was cloned into the pIK6.1 plasmid. The PCR product was generated using primers 5'-tactaacctaggACGCGGTCCCAGATGTTG -3' (Ad5 Nts 10504 to 10521 ) (SEQ ID NO: 12) and 5'-tactaacactacCCGCTGCTCTTGCTCTTG -3' (Ad5 NTs 11095 to 11112) (SEQ ID NO:13). These sequences containing either the Avr II or Dra III site in lowercase were present to facilitate cloning (Figure 13). The cloned virus-associated RNA gene was sequenced to verify the accuracy of the sequence.

The particular embodiments of the invention described above, are, therefore, to be considered as illustrative and not restrictive. The scope of the invention is as set forth in the appended claims rather than being limited to the examples contained in the foregoing description.

## Claims

1. A DNA plasmid comprising an adenoviral gene fragment E4 open reading frame ORF6 operably linked to an inducible promoter.

2. A DNA plasmid according to claim 1, wherein the inducible promoter contains a cAMP response element (CRE).

3. A DNA plasmid according to claim 2, wherein the inducible promoter is regulated by a CRE binding protein.

4. A DNA plasmid according to claim 2, wherein the inducible promoter is a mammalian alpha inhibin promoter.

5. A DNA plasmid according to claim 4, wherein the inducible promoter is a mouse alpha inhibin promoter.

6. A DNA plasmid according to claim 1, wherein the inducible promoter is a drug inducible tetracycline responsive promoter.

7. A DNA plasmid according to Claim 1 which further expresses an adenoviral E2A gene fragment operably linked to an inducible promoter.

8. A DNA plasmid according to claim 1, wherein the plasmid is pIK6.1 MIP(α) -E4 ORF6 designated ATCC # 97325.

9. A DNA plasmid according to claim 1, wherein the plasmid is pIK6.1 MIP(α)-E4 designated ATCC #75879.

10. A packaging cell line derived from a 293 cell that supports the growth of a mutant adenovirus defective in replication or a recombinant adenoviral vector, wherein said adenovirus or adenoviral vector comprises a transgene, a lethal deletion or mutation in each of the E1 early gene region, and an essential region of the E4 early gene region.

11. A packaging cell line according to claim 10, wherein said adenovirus or adenoviral vector further comprises a lethal deletion or mutation in the E2A early gene region.

12. A packaging cell line according to claim 10, wherein said adenovirus or adenoviral vector further comprises a deletion or mutation in the E3 gene region.

13. A packaging cell line according to claim 10, wherein said 293 cells are cells transfected with the adenovirus 5 E4-ORF6 DNA gene fragment designated ATCC CRL# 11990.

14. A packaging cell line according to claim 10 that supplies the function of the E4 early gene region and supports the growth of a mutant adenovirus, wherein the nucleotide sequence encoding the E4 early gene region is operably linked to an inducible promoter.

15. A packaging cell line according to claim 14 that further supplies the function of the E2A early gene region
wherein the nucleotide sequences encoding the E2A and E4 early gene regions are operably linked to an inducible promoter.

16. A packaging cell line according to claims 10, 11 or 15 that supplies the function of the E1, E2A and E4 early gene regions.

17. A packaging cell line according to claim 12 or 15 that further supplies the function of the E3 early gene region.

18. A recombinant adenoviral vector, wherein said vector comprises a transgene, a lethal deletion or mutation in the E1 early gene region, and a lethal deletion or mutation in an essential region of the E4 early gene region, so that when rescued the resulting recombinant adenovirus requires for replication complementation of both the E1 and E4-ORF6 adenoviral early gene regions.

19. A recombinant adenoviral vector according to claim 18 wherein said vector further comprises one or both of a lethal deletion or mutation in the E2A and a lethal deletion or mutation in the E3 early gene regions.

20. A recombinant adenoviral vector according to claims 18 or 19, wherein said transgene encodes a cytokine gene, suicide gene, tumor suppressor gene, protective gene or viral protein.

21. A method of producing a replication-defective adenovirus, said method comprising:
(a) transfecting the packaging cell line of claim 10, 11, 12, 13 or 14 with a recombinant adenoviral vector, said adenoviral vector comprising a transgene and a lethal deletion or mutation in the E4 early gene region and a lethal deletion or mutation in the E1 early gene region; and
(b) rescuing replication-defective adenovirus produced.

22. A method of producing a replication-defective adenovirus, said method comprising:
(a) transfecting the packaging cell line of claim 11, 15 or 16 with a recombinant adenoviral vector, said adenoviral vector comprising a transgene, a lethal deletion or mutation in the E2A early gene region, and a lethal deletion or mutation in the E4 early gene region and the E1 early gene region; and
(b) rescuing replication-defective adenovirus produced.

23. An in vitro method of expressing a transgene in a mammalian target cell, said method comprising the steps of:
(a) infecting said target cell with the replication-defective adenovirus of claim 18, 19 or 20; and
(b) expressing said transgene in said target cell.

24. The method of claim 23, wherein the transgene encodes a cytokine gene, suicide gene, tumor suppressor gene, protective gene or viral protein.

25. A mammalian target cell infected with a replication-defective adenovirus of claim 23.

## Patentansprüche

1. DNA-Plasmid, umfassend einen offenen Leserahmen ORF6 des adenoviralen Genfragments E4, operabel verknüpft mit einem induzierbaren Promotor.

2. DNA-Plasmid nach Anspruch 1, wobei der induzierbare Promotor ein cAMP-Response-Element (CRE) enthält.

3. DNA-Plasmid nach Anspruch 2, wobei der induzierbare Promotor durch ein CRE bindendes Protein reguliert wird.

4. DNA-Plasmid nach Anspruch 2, wobei der induzierbare Promotor ein Säugeralpha-Inhibin-Promotor ist.

5. DNA-Plasmid nach Anspruch 4, wobei der induzierbare Promotor ein Mäusealpha-Inhibin-Promotor ist.

6. DNA-Plasmid nach Anspruch 1, wobei der induzierbare Promotor ein durch Arzneimittel induzierbarer, auf Tetracyclin ansprechender Promotor ist.

7. DNA-Plasmid nach Anspruch 1, welches weiterhin ein adenovirales E2A-Genfragment, operabel verknüpft mit einem induzierbaren Promotor, exprimiert.

8. DNA-Plasmid nach Anspruch 1, wobei das Plasmid pIK6.1 MIP(α) - E4 ORF6, bezeichnet als ATCC # 97325, ist.

9. DNA-Plasmid nach Anspruch 1, wobei das Plasmid pIK6.1 MIP(α) - E4, bezeichnet als ATCC # 75879, ist.

10. Verpackungszellinie, abgeleitet von einer 293-Zelle, die das Wachstum eines mutanten Adenovirus, defekt in der Replikation, oder eines rekombinanten adenoviralen Vektors unterstützt, wobei der Adenovirus oder adenovirale Vektor ein Transgen, eine letale Deletion oder Mutation in jeder von der frühen El-Genregion und einer wesentlichen Region der frühen E4-Genregion umfaßt.

11. Verpackungszellinie nach Anspruch 10, wobei der Adenovirus oder adenovirale Vektor weiterhin eine letale Deletion oder Mutation in der frühen E2A-Genregion umfaßt.

12. Verpackungszellinie nach Anspruch 10, wobei der Adenovirus oder adenovirale Vektor weiterhin eine Deletion oder Mutation in der E3-Genregion umfaßt.

13. Verpaekungszellinie nach Anspruch 10, wobei die 293-Zellen Zellen, transfiziert mit dem E4-ORF6-DNA-Genfragment des Adenovirus 5, bezeichnet als ATCC CRL# 11990, sind.

14. Verpackungszellinie nach Anspruch 10, die die Funktion der frühen E4-Genregion ausfüllt und das Wachstum eines mutanten Adenovirus unterstützt, wobei die Nucleotidsequenz, codierend die frühe E4-Genregion, operabel mit einem induzierbaren Promotor verknüpft ist.

15. Verpackungszellinie nach Anspruch 14, die weiterhin die Funktion der frühen E2A-Genregion ausfüllt, wobei die Nucleotidsequenzen, codierend die frühe E2A- und E4-Genregion, operabel mit einem induzierbaren Promotor verknüpft sind.

16. Verpackungszellinie nach den Ansprüchen 10, 11 oder 15, die die Funktion der frühen E1-, E2A- und E4-Genregion ausfüllt.

17. Verpackungszellinie nach den Ansprüchen 12 oder 15, die weiterhin die Funktion der frühen E3-Genregion ausfüllt.

18. Rekombinanter adenoviraler Vektor, wobei der Vektor ein Transgen, eine letale Deletion oder Mutation in der frühen E1-Genregion und eine letale Deletion oder Mutation in einer wesentlichen Region der frühen E4-Genregion umfaßt, so daß, wenn der resultierende rekombinante Adenovirus gewonnen wird, er zur Replikation Komplementation von sowohl der adenoviralen frühen E1- als auch der adenoviralen frühen E4-ORF6-Genregion erfordert.

19. Rekombinanter adenoviraler Vektor nach Anspruch 18, wobei der Vektor weiterhin eines oder beide von einer letalen Deletion oder Mutation in der frühen E2A-und einer letalen Deletion oder Mutation in der frühen E3-Genregion umfaßt.

20. Rekombinanter adenoviraler Vektor nach den Ansprüchen 18 oder 19, wobei das Transgen ein Cytokin-Gen, Suizid-Gen, Tumorsuppressor-Gen, Schutz-Gen oder virales Protein codiert.

21. Verfahren zum Herstellen eines replikationsdefekten Adenovirus, wobei das Verfahren umfaßt:
(a) Transfizieren der Verpackungszellinie nach Anspruch 10, 11, 12, 13 oder 14 mit einem rekombinanten adenoviralen Vektor, wobei der adenovirale Vektor ein Transgen und eine letale Deletion oder Mutation in der frühen E4-Genregion und eine letale Deletion oder Mutation in der frühen E1-Genregion umfaßt; und
(b) Gewinnen des hergestellten replikationsdefekten Adenovirus.

22. Verfahren zum Herstellen eines replikationsdefekten Adenovirus, wobei das Verfahren umfaßt;
(a) Transfizieren der Verpackungszellinie nach Anspruch 11, 15 oder 16 mit einem rekombinanten adenoviralen Vektor, wobei der adenovirale Vektor ein Transgen, eine letale Deletion oder Mutation in der frühen E2A-Genregion und eine letale Deletion oder Mutation in der frühen E4-Genregion und der frühen El-Genregion umfaßt; und
(b) Gewinnen des hergestellten replikationsdefekten Adenovirus.

23. In-vitro-Verfahren zum Exprimieren eines Transgens in einer Säugerzielzelle, wobei das Verfahren die Schritte umfaßt:
(a) Infizieren der Zielzelle mit dem replikationsdefekten Adenovirus nach Anspruch 18, 19 oder 20; und
(b) Exprimieren des Transgens in der Zielzelle.

24. Verfahren nach Anspruch 23, wobei das Transgen ein Cytokin-Gen, Suizid-Gen, Tumorsuppressor-Gen, Schutz-Gen oder virales Protein codiert.

25. Säugetzielzelle, infiziert mit einem replikationsdefekten Adenovirus nach Anspruch 23.

## Revendications

1. Plasmide ADN comprenant un cadre de lecture ouvert ORF6 d'un fragment de gène adénoviral E4 lié de manière opérationnelle à un promoteur inductible.

2. Plasmide ADN selon la revendication 1, dans lequel le promoteur inductible contient un élément répondant à l'AMPc (CRE).

3. Plasmide ADN selon la revendication 2, dans lequel le promoteur inductible est régulé par une protéine de liaison au CRE.

4. Plasmide ADN selon la revendication 2, dans lequel le promoteur inductible est un promoteur d'alpha-inhibine mammalienne.

5. Plasmide ADN selon la revendication 4, dans lequel le promoteur inductible est un promoteur d'alpha-inhibine de souris.

6. Plasmide ADN selon la revendication 1, dans lequel le promoteur inductible est un promoteur sensible à la tétracycline inductible par un médicament.

7. Plasmide ADN selon la revendication 1, qui exprime en outre un fragment de gène adénoviral E2A lié de manière opérationnelle à un promoteur inductible.

8. Plasmide ADN selon la revendication 1, dans lequel le plasmide est pIK6.1 MIP (α) - E4 ORF6, désigné ATCC # 97325.

9. Plasmide ADN selon la revendication 1, dans lequel le plasmide est pIK6.1 MIP (α) - E4, désigné ATCC #75879.

10. Lignée cellulaire d'encapsidation dérivée d'une cellule 293 qui supporte la croissance d'un adénovirus mutant défectueux en réplication ou un vecteur adénoviral recombinant, où ledit adénovirus ou vecteur adénoviral comprend un transgène, une délétion ou mutation létale dans chacune de la région précoce du gène E1 et d'une région essentielle de la région précoce du gène E4.

11. Lignée cellulaire d'encapsidabion selon la revendication 10, dans laquelle ledit adénovirus ou vecteur adénoviral comprend en outre une délétion ou mutation létale dans la région précoce du gène E2A.

12. Lignée cellulaire d'encapsidation selon la revendication 10, dans laquelle ledit adénovirus ou vecteur adénoviral comprend en outre une délétion ou mutation dans la région du gène E3.

13. Lignée cellulaire d'encapsidation selon la revendication 10, dans laquelle lesdites cellules 293 sont des cellules transfectées avec le fragment du gène ADN E4-ORF6 de l'adénovirus 5 désigné ATCC CRL# 11990.

14. Lignée cellulaire d'encapsidation selon la revendication 10, qui fournit la fonction de la région précoce du gène E4 et supporte la croissance d'un adénovirus mutant, en vertu de quoi la séquence de nucléotides codant la région précoce du gène E4 est liée de manière opérationnelle à un promoteur inductible.

15. Lignée cellulaire d'encapsidation selon la revendication 14, qui fournit en outre la fonction de la région précoce du gène E2A, en vertu de quoi les séquences de nucléotides codant les régions précoces des gènes E2A et E4, sont liées de manière opérationnelle à un promoteur inductible.

16. Lignée cellulaire d'exicapsidation selon les revendications 10, 11 ou 15, qui fournit la fonction des régions précoces des gènes E1, E2A et E4.

17. Lignée cellulaire d'encapsidation selon la revendication 12 ou 15, qui fournit en outre la fonction de la région précoce du gène E3.

18. Vecteur adénoviral recombinant, où ledit vecteur comprend un transgène, une délétion ou mutation létale dans la région précoce du gène E1, et une délétion ou mutation létale dans une région essentielle de la région précoce du gène E4, de sorte que lorsqu'il est secouru l'adénovirus recombinant résultant exige pour la réplication, la complémentation des deux régions précoces des gènes adénoviraux E1 et E4-ORF6.

19. Vecteur adénoviral recombinant selon la revendication 18, dans lequel ledit vecteur comprend en outre l'une ou les deux d'une délétion ou mutation létale dans la région précoce du gène E2A et d'une délétion ou mutation létale dans la région précoce du gène E3.

20. Vecteur adénoviral recombinant selon les revendications 18 ou 19, dans lequel ledit transgène code un gène de cytokine, un gène suicide, un gène suppresscur de tumeur, un gène protecteur ou une protéine virale.

21. Procédé de production d'un adénovirus défectueux à la réplication, ledit procédé comprenant:
(a) transfecter la lignée cellulaire d'encagsidation de la revendication 10, 11, 12, 13 ou 14 avec un vecteur adénoviral recombinant, ledit vecteur adénoviral comprenant un transgène et une délétion ou mutation létale dans la région précoce du gène E4 et une délétion ou mutation létale dans la région précoce du gène E1; et
(b) récupérer l'adénovirus défectueux à la réplication produit.

22. Procédé de production d'un adénovirus défectueux à la réplication, ledit procédé comprenant:
(a) transfecter la lignée cellulaire d'encapsidation de la revendication 11, 15 ou 16 avec un vecteur adénoviral recombinant, ledit vecteur adénoviral comprenant un transgène, une délétion ou mutation létale dans la région précoce du gène E2A et une délétion ou mutation létale dans la région précoce du gène E4 et dans la région précoce du gène E1; et
(b) récupérer l'adénovirus défectueux à la réplication produit.

23. Procédé *in vitro* d'expression d'un transgène dans une cellule cible mammalienne, ledit procédé comprenant les étapes consistant à:
(a) infecter ladite cellule cible avec l'adénovirus défectueux à la réplication de la revendication 18, 19 ou 20; et
(b) exprimer ledit transgène dans ladite cellule cible.

24. Le procédé de la revendication 23, dans lequel le transgène code un gène de cytokine, un gène suicide, un gène supprcsseur de tumeur, un gène protecteur ou une protéine virale.

25. Cellule cible mammalienne infectée avec un adénovirus défectueux à la réplication de la revendication 23.


### SEQUENCE LISTING

(1) GENERAL INFORMATION:
   (i) APPLICANT: WANG, QING FINER, MITCHELL H. JIA, XIAO-CHI
   (ii) TITLE OF INVENTION: NOVEL ADENOVIRAL VECTORS, PACKAGING CELL LINES, RECOMBINANT ADENOVIRUSES, AND METHODS
   (iii) NUMBER OF SEQUENCES: 13
   (iv) CORRESPONDENCE ADDRESS:
      (A) ADDRESSEE: CELL GENESYS, INC.
      (B) STREET: 322 LAKESIDE DRIVE
      (C) CITY: FOSTER CITY
      (D) STATE: CALIFORNIA
      (E) COUNTRY: USA
      (F) ZIP: 94404
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE: 03-NOV-1995
      (C) CLASSIFICATION:
   (viii) ATTORNEY/AGENT INFORMATION:
      (A) NAME: KRUPEN, KAREN I.
      (B) REGISTRATION NUMBER: 34,647
      (C) REFERENCE/DOCKET NUMBER: CELL 16.3
   (ix) TELECOMMUNICATION INFORMATION:
      (A) TELEPHONE: (415)358-9600 X131
      (B) TELEFAX: (415)349-3792
(2) INFORMATION FOR SEQ ID NO:1:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
   GCGCAAGCTT CGGGAGTGGG AGATAAGGCT C 31
(2) INFORMATION FOR SEQ ID NO:2:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
   GGCCTCTAGA AGTTCACTTG CCCTGATGAC A 31
(2) INFORMATION FOR SEQ ID NO:3:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
   GAGGACTAAG GATTGATT 18
(2) INFORMATION FOR SEQ ID NO:4:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
   CGTGAGATTT TGGATAAG 18
(2) INFORMATION FOR SEQ ID NO:5:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   CCTACGCACG AC 12
(2) INFORMATION FOR SEQ ID NO:6:
   (i) SEQUENCE CHARACTERISTICS:
      (A) LENGTH:. 12 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
   GACCGTGAGG ATACT
   15
(2) INFORMATION FOR SEQ ID NO:8:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   GCCAATCTAG AGCTTCAGGA AATATGACT
   29
(2) INFORMATION FOR SEQ ID NO:9:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
   CATCTCTCGA GGGAGAAGTC CACGCCTAC
   29
(2) INFORMATION FOR SEQ ID NO:10:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DHA (genomic)
   (xi) SEQUENCE DESCRIPTION: SEQ ID NO:10:
   TCCATTTCTA GATCGGCTGC GGTTG
   25
(2) INFORMATION FOR SEQ ID NO:11:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
   ACGTGGTACT TGTCCATC
   18
(2) INFORMATION FOR SEQ ID NO:12:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
   TACTAACACT ACCCGCTGCT CTTGCTCTTG
   30
(2) INFORMATION FOR SEQ ID NO:13:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13: